# EUROPEAN PATENT APPLICATION

(11) **EP 2 158 837 A2**
(43) Date of publication of application: **03.03.2010**
(21) Application number: 09011021.4
(22) Date of filing: 27.08.2009
(51) Int. Cl.: A61B 1/31, A61M 25/01

(54) **Actuator for intraductal moving body and endoscope**

(30) Priority: 28.08.2008 JP 2008219592; 02.09.2008 JP 2008224704; 17.09.2008 JP 2008238281
(71) Applicant: Fujifilm Corporation, Tokyo 106-8620 (JP)
(72) Inventor: Nagamachi, Toshiharu, Ashigarakami-gun Kanagawa 258-8538 (JP); Iida, Takayuki, Ashigarakami-gun Kanagawa 258-8538 (JP)
(74) Representative: Klunker . Schmitt-Nilson . Hirsch

(57) **Abstract**

To bring a propulsion mechanism into reliable contact with a duct wall and transfer just a propulsion force to the duct wall without transferring an unnecessary retraction operation. An actuator for intraductal moving body includes: a propulsion mechanism (32, 36, 54, 56) which generates a drive force via a duct wall to generate a propulsion force for movement in a duct; a base mechanism (30, 34, 80) which supports the propulsion mechanism and causes the propulsion mechanism to abut against and separate from the duct wall; and two or more pairs of bilayer mechanisms provided axially symmetrically.

## Description

### BACKGROUND OF THE INVENTION

### Field of the Invention

The present invention relates to an intraductal moving body actuator and an endoscope, and more particularly to a technique of transferring a propulsion force to a duct wall and moving in a duct.

### Description of the Related Art

Japanese Patent Application Laid-Open No. 2008-43669 discloses an intraductal moving body actuator that moves in a duct. Specifically, a balloon is mounted to an insertion portion of an electronic endoscope, and the balloon is configured so that a rear portion in an advancing direction and a circumferential portion have a lower expansion coefficient than other portions. Air is supplied to expand the balloon rearward in the advancing direction, thus a surface of the balloon in contact with an inner wall surface of the duct generates a drive force rearward in the advancing direction via the inner wall surface while being in contact with the inner wall surface, and this force moves the insertion portion in the advancing direction.

Japanese Patent Application Laid-Open No. 2000-230510 discloses an actuator body formed of an elastic member having a plurality of pressure chambers formed therein, and having a plurality of protrusions formed on an upper surface. A diaphragm between adjacent pressure chambers is formed into a bellows shape. Thus, an inside pressure of each chamber is increased and reduced to vertically significantly extend and contract the diaphragm to increase amplitude of elastic deformation waves, thereby preventing stop of movement/conveyance even with irregularities on a movement surface of the actuator or a contact surface of a conveyed object.

Japanese Patent Application Laid-Open No. 5-293077 discloses an intraductal insertion device including, at a distal end of an insertion portion, an axially extendable and contractable bellows, a first balloon and a second balloon provided at opposite ends of the bellows and lockable (grippable) on an inner peripheral wall of a lumen so as to hold their positions, and a suction device that is provided in at least one of the balloons, and is opened into the inner peripheral wall of the lumen to apply negative pressure to the inner peripheral wall.

In the intraductal insertion device in Japanese Patent Application Laid-Open No. 5-293077, the second balloon is expanded, and a space chamber is sucked from a second suction duct to ensure grip on the inner peripheral wall of the lumen, and then pressure is applied to the inside of the bellows to move the first balloon forward and thus move the distal end portion forward.

### SUMMARY OF THE INVENTION

For reliable contact of a lock balloon for locking on an intestinal wall radially elastically deformed or a propulsion mechanism for propulsion, a diameter of an outer peripheral surface of the lock balloon or the propulsion mechanism during propulsion needs to be about three times larger than a diameter of an endoscope. However, in the devices described in Japanese Patent Application Laid-Open Nos. 2008-43669, 2000-230510, and 5-293077, a propulsion force cannot be generated when the balloon surface or the protrusion does not come into contact with (does not reach) an intestinal wall.

Further, even if the balloon surface or the protrusion comes into contact with the intestinal wall, there is a possibility that the intestine is merely deformed without the propulsion force being exerted. Specifically, in Japanese Patent Application Laid-Open No. 2008-43669, when air is released to contract the balloon after expansion, the balloon is contracted forward in the advancing direction, and thus as a reverse action from the action during expansion of the balloon, the surface of the balloon in contact with the inner wall surface of the duct generates a retraction force forward in the advancing direction via the inner wall surface while being in contact with the inner wall surface, and this force transfers an unnecessary retraction movement to the duct wall, thereby preventing a significant advance amount from being obtained.

In Japanese Patent Application Laid-Open No. 2000-230510, there is a possibility that the surface of the intestine merely contracts synchronously with the protrusions and that the propulsion force cannot be exerted.

Also, in a self-propelled system with a double balloon, a distal end portion of an endoscope only has a locking function, and a pushing operation is performed with a rear balloon as a starting point, and thus an extension and contraction mechanism of a self-propelled portion is sometimes stuck in a bent part of an intestinal tract and cannot advance.

The present invention is achieved in view of these circumstances, and has an object to provide an intraductal moving body actuator and an endoscope that can reliably bring a propulsion mechanism into contact with a duct wall and reliably transfer just a propulsion force to the duct wall without transferring an unnecessary retraction operation.

In a self-propelled system with a double balloon as in Japanese Patent Application Laid-Open No. 5-293077, the first balloon and the second balloon at the distal end portion of the endoscope only has a locking function. Since a pushing operation is performed by expansion and contraction of the bellows, an extension and contraction mechanism is sometimes stuck in a bent part of an intestinal tract to prevent advance.

Also, when a plurality of balloons are provided, providing pressure sources corresponding to the plurality of balloons increases cost and size of the device.

The present invention is achieved in view of these circumstances, and has an object to provide an intraductal moving body actuator and an endoscope that can reliably bring a propulsion mechanism into contact with a duct wall and reliably transfer just a propulsion force to a duct wall without transferring an unnecessary retraction operation, and reduce the number of drive sources of a base mechanism and the propulsion mechanism to reduce cost and size.

To achieve the objects, a first aspect of the present invention provides an actuator for intraductal moving body, comprising: a propulsion mechanism which generates a drive force via a duct wall to generate a propulsion force for movement in a duct; a base mechanism which supports the propulsion mechanism and causes the propulsion mechanism to abut against and separate from the duct wall; and two or more pairs of bilayer mechanisms provided axially symmetrically.

According to the first aspect of the present invention, the base mechanism causes the propulsion mechanism to reliably abut against the duct wall, thereby allowing a propulsion force from the propulsion mechanism to be reliably transferred to the duct wall.

According to a second aspect of the present invention, the actuator for intraductal moving body further comprises the a control unit which performs control to make the propulsion mechanism generate the propulsion force after the propulsion mechanism is caused to abut against the duct wall by the base mechanism, and to make the base mechanism separate the propulsion mechanism from the duct wall while the propulsion mechanism generating the propulsion force.

Thus, the propulsion mechanism is separated from the duct wall with the propulsion force being generated, thereby allowing just the propulsion force to be reliably transferred to the duct wall without transferring an unnecessary retraction operation.

According to a third aspect of the present invention, in the actuator for intraductal moving body, the base mechanism includes balloons, and the balloons are expanded and contracted to cause the propulsion mechanism to be abut against and separate from the duct wall.

Thus, using the balloon, the propulsion mechanism can reliably abut against and separate from the duct wall with a simple structure.

According to a fourth aspect of the present invention, in the actuator for intraductal moving body, the propulsion mechanism includes balloons, and each of the balloons comprises a portion having a lower expansion coefficient than other portions is provided at least in a rear portion in an advancing direction.

Thus, the balloons can expand rearward in the advancing direction to generate a drive force via the duct wall, thereby allowing an intraductal moving body to be moved in the advancing direction.

According to a fifth aspect of the present invention, in the actuator for intraductal moving body, the base mechanism causes the balloons in a contracted state to abut against the duct wall, and then the base mechanism causes the balloons in an expanded state to separate from the duct wall.

Thus, the balloons can abut against the duct wall in the contracted state, while the balloons can be separated from the duct wall in the expanded state to provide hysteresis to the state of the balloon, thereby allowing just the propulsion force to be reliably transferred to the duct wall without transferring an unnecessary retraction operation.

A sixth aspect of the present invention, provides an endoscope having a function of self-propelled movement in a duct and including the actuator for intraductal moving body according to any one of the first to fifth aspects.

To achieve the objects, a seventh aspect of the present invention provides an actuator for intraductal moving body, comprising: a propulsion mechanism which generates a drive force via a duct wall to generate a propulsion force for movement in a duct; a base mechanism which supports the propulsion mechanism and causes the propulsion mechanism to abut against and separate from the duct wall; a pressure source connected to the base mechanism; and a valve which is provided between the propulsion mechanism and the base mechanism, the valve which is closed until a first pressure difference value is reached and is opened when the first pressure difference value is reached in the case where a pressure in the base mechanism becomes positive with respect to the propulsion mechanism and pressure difference between the propulsion mechanism and the base mechanism is increased by the pressure source, and which is closed until a second pressure difference value is reached and is opened when the second pressure difference value is reached in the case where the pressure in the base mechanism becomes negative with respect to the propulsion mechanism and pressure difference between the propulsion mechanism and the base mechanism is increased by the pressure source.

Thus, the pressure source connected to the base mechanism controls pressure of both the propulsion mechanism and the base mechanism, thereby reducing cost and size of the intraductal moving body actuator.

According to an eighth aspect of the present invention, the actuator for intraductal moving body may further comprise a control unit which performs control to make the propulsion mechanism generate the propulsion force after the propulsion mechanism is caused to abut against the duct wall by the base mechanism, and to make the base mechanism separate the propulsion mechanism from the duct wall while the propulsion mechanism generating the propulsion force.

Thus, the base mechanism causes the propulsion mechanism to be reliably abutted against the duct wall, thereby allowing a propulsion force from the propulsion mechanism to be reliably transferred to the duct wall.

Also, the propulsion mechanism is separated from the duct wall with the propulsion force being generated, thereby allowing just the propulsion force to be reliably transferred to the duct wall without transferring an unnecessary retraction operation.

According to a ninth aspect of the present invention, in the actuator for intraductal moving body, the base mechanism includes balloons, and the balloons are expanded and contracted to cause the propulsion mechanism to be abut against and separate from the duct wall.

Thus, using the balloons, the propulsion mechanism can abut against and separate from the duct wall with a simple structure.

According to a tenth aspect of the present invention, in the actuator for intraductal moving body, the propulsion mechanism includes balloons, and each of the balloons comprises a portion having a lower expansion coefficient than other portions is provided at least in a rear portion in an advancing direction.

Thus, the balloons can expand rearward in the advancing direction to generate a drive force via the duct wall, thereby allowing an intraductal moving body to be moved in the advancing direction.

According to an eleventh aspect of the present invention, in the actuator for intraductal moving body, the base mechanism causes the balloons in a contracted state to abut against the duct wall, and then the base mechanism causes the balloons in an expanded state to separate from the duct wall.

Thus, the balloons can abut against the duct wall in the contracted state, while the balloons are separated from the duct wall in the expanded state to provide hysteresis to the state of the balloon, thereby allowing just the propulsion force to be reliably transferred to the duct wall without transferring an unnecessary retraction operation.

To achieve the objects, a twelfth aspect of the present invention provides an endoscope having a function of self-propelled movement in a duct and including the actuator for intraductal moving body according to any one of the seventh to eleventh aspects.

To achieve the objects, a thirteenth aspect of the present invention provides an actuator for intraductal moving body, comprising: a propulsion mechanism having a first region and a pair of second regions connected to opposite ends of the first region and applying a propulsion force to a duct wall; and a control unit which performs control so that one of the second regions is changed from a contracted state to an extended state and the other of the second regions is changed from the extended state to the contracted state with the first region abutting against the duct wall, in order to apply the propulsion force with respect to the duct wall to the first region applies.

Thus, the propulsion mechanism applies the propulsion force to the duct wall to move the intraductal moving body actuator relative to the duct wall.

According to a fourteenth aspect of the present invention, in the actuator for intraductal moving body, the control unit controls to maintain the one of the second regions in the extended state and maintain the other of the second regions in the contracted state in order to make the first region separate from the duct wall while a state where the propulsion force to the duct wall is applied to the first region being maintained.

Thus, an unnecessary retraction operation is not transferred to the duct wall, and a position after the movement of the intraductal moving body actuator is maintained.

According to a fifteenth aspect of the present invention, in the actuator for intraductal moving body, the one of the second regions, the first region, and the other of the second regions are arranged in order in a movement direction.

Thus, the intraductal moving body actuator can be moved relative to the duct wall in a desired movement direction.

According to a sixteenth aspect of the present invention, in the actuator for intraductal moving body, the second region includes shape memory material or artificial muscle.

Thus, an extension function and a contraction function of the shape memory material or the artificial muscle can be used to extend and contract the second region.

According to a seventeenth aspect of the present invention, in the actuator for intraductal moving body, the second region includes an outer peripheral portion of a pressure chamber, and the control unit controls to change an internal pressure of the pressure chamber to extend or contract the second region.

Thus, the internal pressure of the pressure chamber can be changed to extend and contract the second region.

According to an eighteenth aspect of the present invention, the actuator for intraductal moving body further includes a locking mechanism which locks on the duct wall.

Thus, the locking of the locking mechanism on the duct wall allows the intraductal moving body actuator to be reliably moved relative to the duct wall.

According to a nineteenth aspect of the present invention, in the actuator for intraductal moving body, the locking mechanism includes the first region and the pair of second regions.

Thus, the locking of the locking mechanism on the duct wall allows the intraductal moving body actuator to be reliably moved relative to the duct wall.

According to a twentieth aspect of the present invention, the actuator for intraductal moving body further includes a base mechanism which supports the propulsion mechanism and causes the propulsion mechanism to abut against and separate from the duct wall.

Thus, the base mechanism can cause the propulsion mechanism to be reliably abutted against and separated from the duct wall, and the propulsion mechanism can more reliably apply a propulsion force to the duct wall to move the intraductal moving body actuator relative to the duct wall.

To achieve the objects, a twenty first aspect of the present invention provides an endoscope having a function of self-propelled movement in a duct and including an intraductal moving body actuator according to any one of the thirteenth to twentieth aspects.

The present invention can bring a propulsion mechanism into reliable contact with a duct wall and reliably transfer just a propulsion force to the duct wall without transferring an unnecessary retraction operation.

The present invention can bring a propulsion mechanism into reliable contact with a duct wall and reliably transfer just a propulsion force to the duct wall without transferring an unnecessary retraction operation, and reduce the number of drive sources of a base mechanism and the propulsion mechanism to reduce cost and size.

### BRIEF DESCRIPTION OF THE DRAWINGS

Fig. 1 is a configuration diagram of an electronic endoscope;
Figs. 2A and 2B illustrate a configuration of an intraductal moving body actuator at a distal end portion of an insertion portion;
Fig. 3 is an enlarged view of a first propulsion balloon;
Fig. 4 is a block diagram of a balloon control device;
Figs. 5A and 5B are a flowchart of a specific propulsion operation of the intraductal moving body actuator of the present invention;
Figs. 6A to 6J are schematic views of expansion and contraction of each balloon;
Fig. 7 is a timing chart illustrating changes with time of expansion and contraction (pressure) of each balloon;
Figs. 8A to 8D illustrate parts of a propulsion operation of a variant;
Fig. 9 is a configuration diagram of an endoscope moving device in use;
Fig. 10 is a configuration diagram of an elastic member as a variant of a propulsion mechanism;
Fig. 11A is a layout view and Fig. 11B is a configuration diagram of a lift mechanism as a variant of a base mechanism;
Fig. 12 is an enlarged view of a first propulsion balloon;
Fig. 13 is a block diagram of a relationship between a balloon control device, each balloon, a first valve, and a second valve;
Fig. 14 is a schematic view of a configuration of a first lift-up balloon, a first propulsion balloon, a first valve, and a first pump;
Figs. 15A to 15D are a flowchart of a specific propulsion operation of the intraductal moving body actuator of the present invention;
Fig. 16A is a schematic view of expansion and contraction of each balloon;
Fig. 16B is a schematic view of expansion and contraction of each balloon;
Fig. 16C is a schematic view of expansion and contraction of each balloon;
Fig. 16D is a schematic view of expansion and contraction of each balloon;
Fig. 16E is a schematic view of expansion and contraction of each balloon;
Fig. 16F is a schematic view of expansion and contraction of each balloon;
Fig. 16G is a schematic view of expansion and contraction of each balloon;
Fig. 16H is a schematic view of expansion and contraction of each balloon;
Fig. 16I is a schematic view of expansion and contraction of each balloon;
Fig. 17 is a timing chart illustrating changes with time of expansion and contraction (pressure) of each balloon;
Fig. 18A is a schematic view of expansion and contraction of the first lift-up balloon and the first propulsion balloon and opening/closing of the first valve;
Fig. 18B is a schematic view of expansion and contraction of the first lift-up balloon and the first propulsion balloon and opening/closing of the first valve;
Fig. 18C is a schematic view of expansion and contraction of the first lift-up balloon and the first propulsion balloon and opening/closing of the first valve;
Fig. 18D is a schematic view of expansion and contraction of the first lift-up balloon and the first propulsion balloon and opening/closing of the first valve;
Fig. 18E is a schematic view of expansion and contraction of the first lift-up balloon and the first propulsion balloon and opening/closing of the first valve;
Fig. 18F is a schematic view of expansion and contraction of the first lift-up balloon and the first propulsion balloon and opening/closing of the first valve;
Fig. 18G is a schematic view of expansion and contraction of the first lift-up balloon and the first propulsion balloon and opening/closing of the first valve;
Fig. 19 illustrates a balloon including three or more layers;
Figs. 20A to 20D illustrate parts of a propulsion operation of a variant;
Fig. 21 is a configuration diagram of an endoscope moving device in use;
Figs. 22A and 22B are block diagrams of an electronic endoscope;
Fig. 23 is an enlarged sectional view of a propulsion balloon as a propulsion mechanism in Example 1 at a distal end portion of an insertion portion;
Fig. 24 is a block diagram of a balloon control device that controls pressure of the propulsion balloon and a lock balloon in Example 1;
Fig. 25 is a flowchart of a detailed propulsion operation in Example 1 of the intraductal moving body actuator of the present invention;
Fig. 26A is a schematic sectional view of expansion and contraction of each balloon associated with the flowchart of the propulsion operation in Fig. 25;
Fig. 26B is a schematic sectional view of expansion and contraction of each balloon associated with the flowchart of the propulsion operation in Fig. 25;
Fig. 26C is a schematic sectional view of expansion and contraction of each balloon associated with the flowchart of the propulsion operation in Fig. 25;
Fig. 26D is a schematic sectional view of expansion and contraction of each balloon associated with the flowchart of the propulsion operation in Fig. 25;
Fig. 26E is a schematic sectional view of expansion and contraction of each balloon associated with the flowchart of the propulsion operation in Fig. 25;
Fig. 26F is a schematic sectional view of expansion and contraction of each balloon associated with the flowchart of the propulsion operation in Fig. 25;
Fig. 26G is a schematic sectional view of expansion and contraction of each balloon associated with the flowchart of the propulsion operation in Fig. 25;
Fig. 26H is a schematic sectional view of expansion and contraction of each balloon associated with the flowchart of the propulsion operation in Fig. 25;
Fig. 27 is an enlarged sectional view of a propulsion balloon as a propulsion mechanism in Example 2 at a distal end portion of an insertion portion;
Fig. 28 is a block diagram of a balloon control device for controlling pressure of the propulsion balloon and a lock balloon in Example 2;
Fig. 29 is a flowchart of a specific propulsion operation in Example 2 of the intraductal moving body actuator of the present invention;
Fig. 30A is a schematic sectional view of expansion and contraction of each balloon associated with the flowchart of the propulsion operation in Fig. 29;
Fig. 30B is a schematic sectional view of expansion and contraction of each balloon associated with the flowchart of the propulsion operation in Fig. 29;
Fig. 30C is a schematic sectional view of expansion and contraction of each balloon associated with the flowchart of the propulsion operation in Fig. 29;
Fig. 30D is a schematic sectional view of expansion and contraction of each balloon associated with the flowchart of the propulsion operation in Fig. 29;
Fig. 30E is a schematic sectional view of expansion and contraction of each balloon associated with the flowchart of the propulsion operation in Fig. 29;
Fig. 30F is a schematic sectional view of expansion and contraction of each balloon associated with the flowchart of the propulsion operation in Fig. 29;
Fig. 30G is a schematic sectional view of expansion and contraction of each balloon associated with the flowchart of the propulsion operation in Fig. 29;
Fig. 30H is a schematic sectional view of expansion and contraction of each balloon associated with the flowchart of the propulsion operation in Fig. 29;
Fig. 31 is a timing chart illustrating changes with time of pressure in each balloon in the propulsion operation in Fig. 29;
Figs. 32A and 32B illustrate a configuration of an intraductal moving body actuator in Example 3 at a distal end portion of an insertion portion;
Fig. 33 is a block diagram of a balloon control device in Example 3;
Figs. 34A and 34B are a flowchart of a specific propulsion operation in Example 3 of the intraductal moving body actuator of the present invention;
Fig. 35A is a schematic view of expansion and contraction of each balloon associated with the flowchart of the propulsion operation in Figs. 34A and 34B;
Fig. 35B is a schematic view of expansion and contraction of each balloon associated with the flowchart of the propulsion operation in Figs. 34A and 34B;
Fig. 35C is a schematic view of expansion and contraction of each balloon associated with the flowchart of the propulsion operation in Figs. 34A and 34B;
Fig. 35D is a schematic view of expansion and contraction of each balloon associated with the flowchart of the propulsion operation in Figs. 34A and 34B;
Fig. 35E is a schematic view of expansion and contraction of each balloon associated with the flowchart of the propulsion operation in Figs. 34A and 34B;
Fig. 35F is a schematic view of expansion and contraction of each balloon associated with the flowchart of the propulsion operation in Figs. 34A and 34B;
Fig. 35G is a schematic view of expansion and contraction of each balloon associated with the flowchart of the propulsion operation in Figs. 34A and 34B;
Fig. 36 is a timing chart illustrating changes with time of pressure in each balloon in the propulsion operation in Figs. 34A and 34B;
Fig. 37 illustrates an example application to an endoscope moving device; and
Figs. 38A and 38B illustrate an example using a lift mechanism as a base mechanism in Example 3.

### DETAILED DESCRIPTION OF THE PREFERRED EMBODIMENTS

Now, preferred embodiments of the present invention will be described with reference to the accompanying drawings.

### [First embodiment]

### [Description on electronic endoscope]

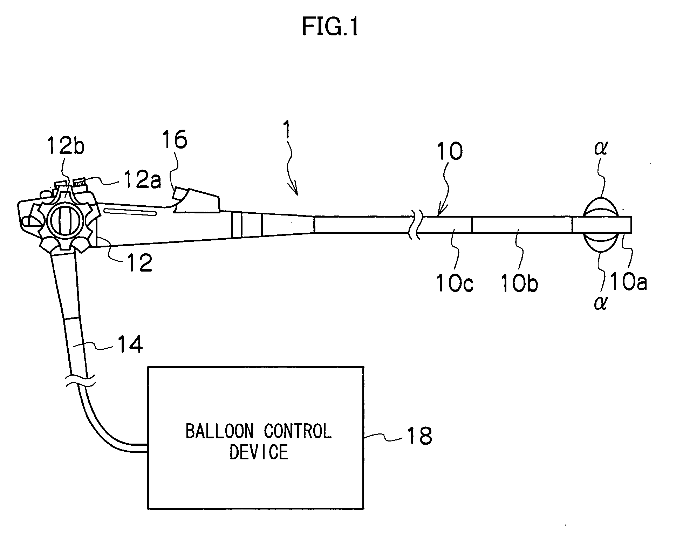

In Fig. 1, an electronic endoscope 1 includes an insertion portion 10 to be inserted into a subject, and an operation portion 12 connected to a proximal end portion of the insertion portion 10. A distal end portion 10a (for example, having an outer diameter of 12 mmφ) connected to a distal end of the insertion portion 10 has a built-in objective lens for capturing an image light of an observed region in the subject, and a built-in imaging device that picks up an image of the image light (both not shown). The image of the inside of the subject obtained by the imaging device is displayed as an endoscope image on a monitor of a processor device (both not shown) connected to a cord 14.

The distal end portion 10a also includes an illumination window for applying illumination light from a light source device (not shown) to the observed region, a forceps exit communicating with a forceps opening 16, and a nozzle for spraying cleaning water or air for cleaning an observation window for protecting the objective lens by operating an air/water supply button 12a.

A bending portion 10b to which a plurality of bending pieces are connected is provided behind the distal end portion 10a. The bending portion 10b vertically and laterally bends by an angle knob 12b provided on the operation portion 12 being operated to push and pull a wire inserted through the insertion portion 10. Thus, the distal end portion 10a is oriented in a desired direction in the subject.

A flexible portion 10c is provided behind the bending portion 10b. The flexible portion 10c has a length of one to several meters so that the distal end portion 10a can reach the observed region and an operator can keep a proper distance from a patient so that the operator may grip and operate the operation portion 12 without any trouble.

To the distal end portion 10a, bilayer structure balloons α and β described later (in Fig. 1, bilayer structure balloons α only are shown) are mounted. As described later, a pair of bilayer structure balloons is arranged opposite to each other circumferentially of the insertion portion 10, that is, 180° apart, and made of, for example, expandable and contractable latex rubber.

Two pairs of bilayer structure balloons are connected to a balloon control device 18 for controlling pressure in each balloon.

When an inner wall surface of a duct bent in a complex manner such as a large intestine or a small intestine is observed with the electronic endoscope 1 configured as described above, the insertion portion 10 is inserted into the subject with the two pairs of bilayer structure balloons α and β being contracted, the light source device is lit to illuminate the inside of the subject, and an endoscope image obtained by the imaging device is observed by the monitor.

When the distal end portion 10a reaches the duct, the balloon control device 18 controls expansion and contraction of the two pairs of bilayer structure balloons α and β to generate a drive force via the inner wall surface of the duct, and the force moves the insertion portion 10 in an advancing direction.

A propulsion operation flow will be described in detail later.

### [Description on intraductal moving body actuator]

Next, an intraductal moving body actuator will be described.

### (Example 1)

### <Configuration and operation of intraductal moving body actuator>

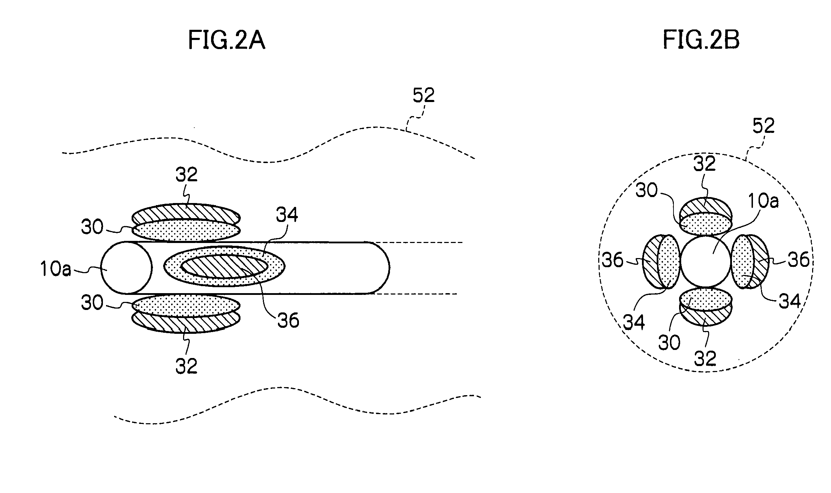

Figs. 2A and 2B illustrate a configuration of the intraductal moving body actuator at the distal end portion 10a of the insertion portion 10. Fig. 2A is a schematic view seen from a lateral side of the distal end portion 10a, and Fig. 2B is a view seen from a distal end side of the distal end portion 10a.

As illustrated in Figs. 2A and 2B, the intraductal moving body actuator includes first lift-up balloons 30 that are provided on an outer peripheral surface of the distal end portion 10a of the electronic endoscope 1 and lifts up first propulsion balloons 32 described below, and the first propulsion balloons 32 provided to overlap an upper side (outer peripheral side) of the first lift-up balloons 30.

The first lift-up balloons 30 serve as a base mechanism that supports the first propulsion balloons 32 and cause the first propulsion balloons 32 to abut against and separate from the duct wall such as an intestinal wall. The first propulsion balloons 32 serve as a propulsion mechanism that generates a drive force via the duct wall such as the intestinal wall to generate a propulsion force for movement in a duct.

An interface between the first lift-up balloon 30 and the first propulsion balloons 32 is connected by bonding or integral molding.

The pair of bilayer structure balloons α as bilayer mechanisms each including the first lift-up balloon 30 and the first propulsion balloon 32 are arranged axially symmetrically at the distal end portion 10a. In the embodiment, a pair of bilayer structure balloons β as bilayer mechanisms each including a second lift-up balloon 34 and a second propulsion balloon 36 are arranged axially symmetrically at the distal end portion 10a of the endoscope in circumferentially 90° shifted positions on the distal end portion 10a. Thus, two pairs of bilayer structure balloons α and β are provided, in total. A total of three or more pairs of bilayer structure balloons may be provided.

In the embodiment, the pair of bilayer structure balloons α and the pair of bilayer structure balloons β are provided substantially in the same axial positions on the distal end portion 10a of the electronic endoscope 1, but the pair of bilayer structure balloons α and the pair of bilayer structure balloons β may be provided in axially shifted positions.

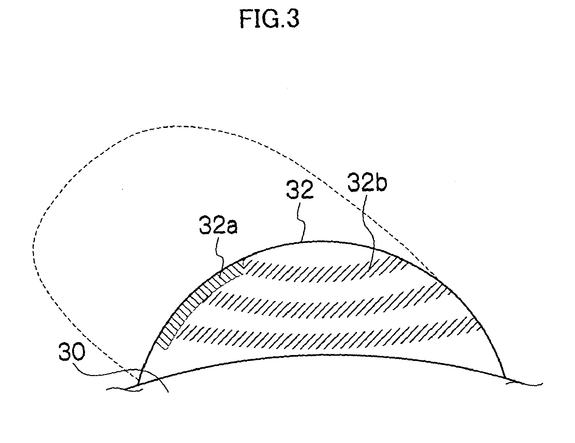

Fig. 3 is an enlarged view of a portion of the first propulsion balloons 32. Each of the second propulsion balloons 36 has the same configuration as the first propulsion balloons 32. The first propulsion balloons 32 are made of expandable and contractable latex rubber, for example.

As illustrated in Fig. 3, in each of the first propulsion balloons 32, a rear portion 32a (shaded portion) in the advancing direction of the insertion portion 10 (a direction toward the distal end portion 10a from a proximal end portion of the insertion portion 10) and a circumferential portion 32b (shaded portion) are thicker than other portions.

Thus, when gas is supplied into the first propulsion balloons 32, each of the first propulsion balloons 32 expands as shown by the dotted line in Fig. 3. Specifically, the rear portion 32a and the circumferential portion 32b are thicker than other portions, and thus the rear portion 32a and the circumferential portion 32b have a lower expansion coefficient than other portions, other portions expand more than the rear portion 32a and the circumferential portion 32b, and each of the first propulsion balloon 32 expands substantially in a fan shape rearward in the advancing direction as shown by the dotted line.

Fig. 3 illustrates an example in which the rear portion 32a and the circumferential portion 32b are thicker than other portions, but the rear portion 32a only may be thicker than other portions.

Low expansion coefficient material (for example, PET fiber or aramid fiber) having a lower expansion coefficient than material for the first propulsion balloon 32 or a coil-shaped wire of shape memory alloy may be joined to, embedded into, or formed integrally with surfaces of the rear portion 32a and the circumferential portion 32b or a surface of the rear portion 32a only to partially change the expansion coefficient.

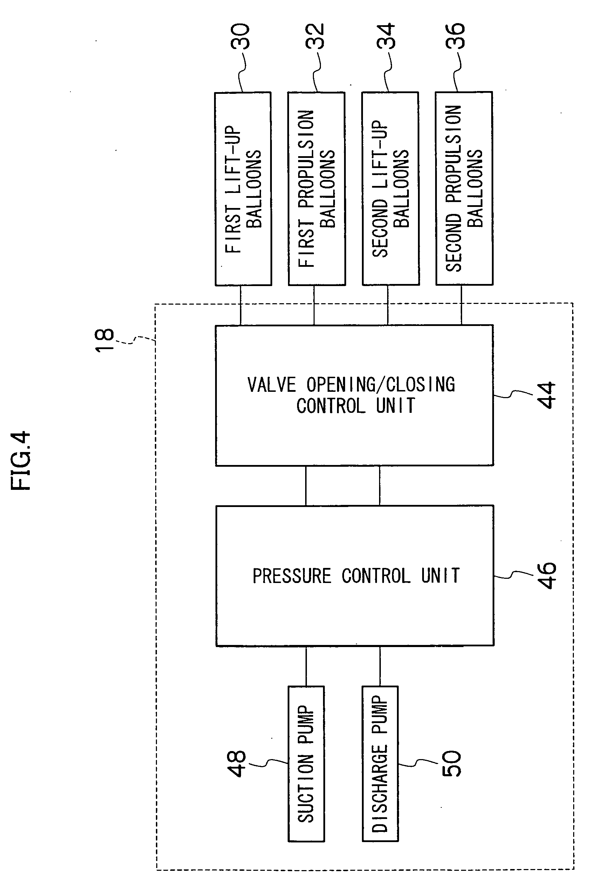

Fig. 4 is a block diagram of the balloon control device 18 for controlling pressure in the first lift-up balloons 30, the first propulsion balloons 32, the second lift-up balloons 34, and the second propulsion balloons 36. As illustrated in Fig. 4, the first lift-up balloons 30, the first propulsion balloons 32, the second lift-up balloons 34, and the second propulsion balloons 36 can be independently adjusted in internal pressure, and a suction pump 48 and a discharge pump 50 are connected to the balloons via a valve opening/closing control unit 44 and a pressure control unit 46.

The propulsion operation flow described below is performed by the valve opening/closing control unit 44 controlling opening/closing of a valve (not shown) connected to each balloon, and the pressure control unit 46 controlling the suction pump 48 and the discharge pump 50.

### <Propulsion operation flow>

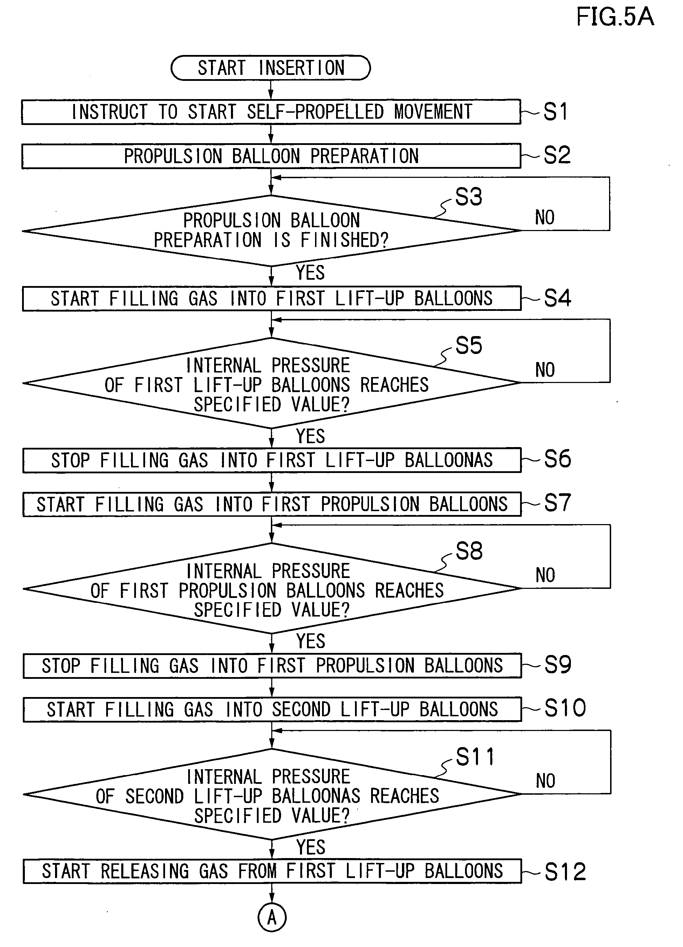

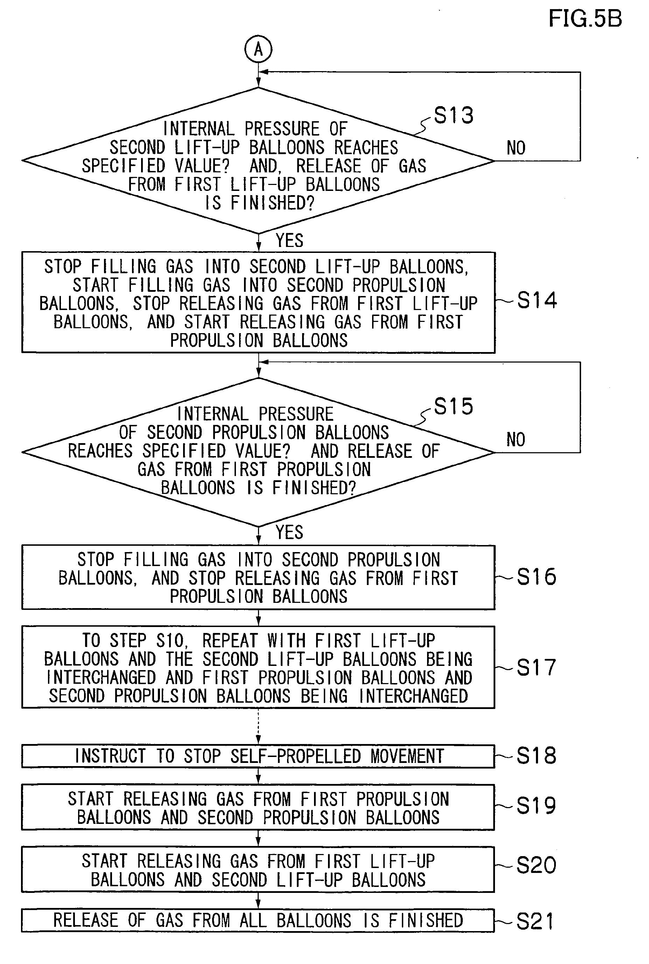

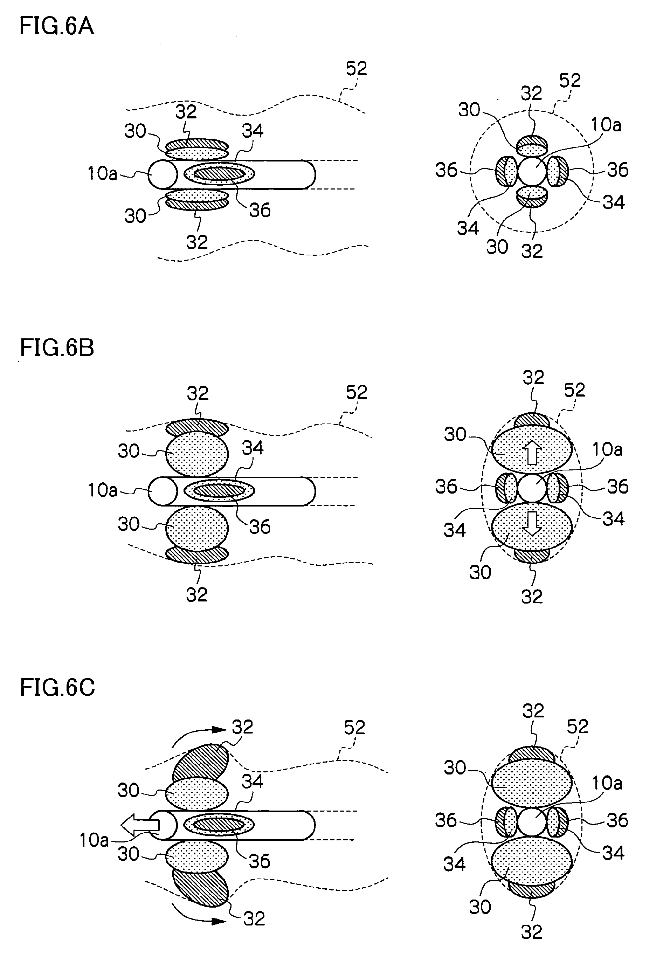

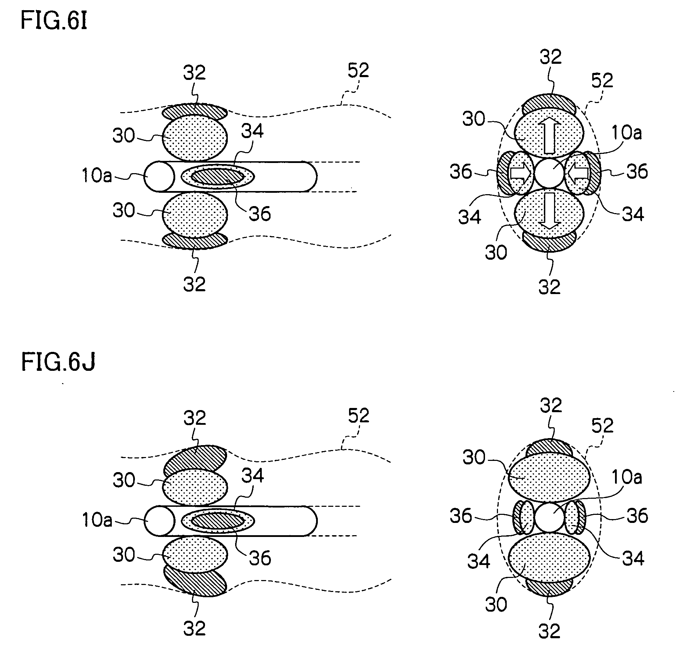

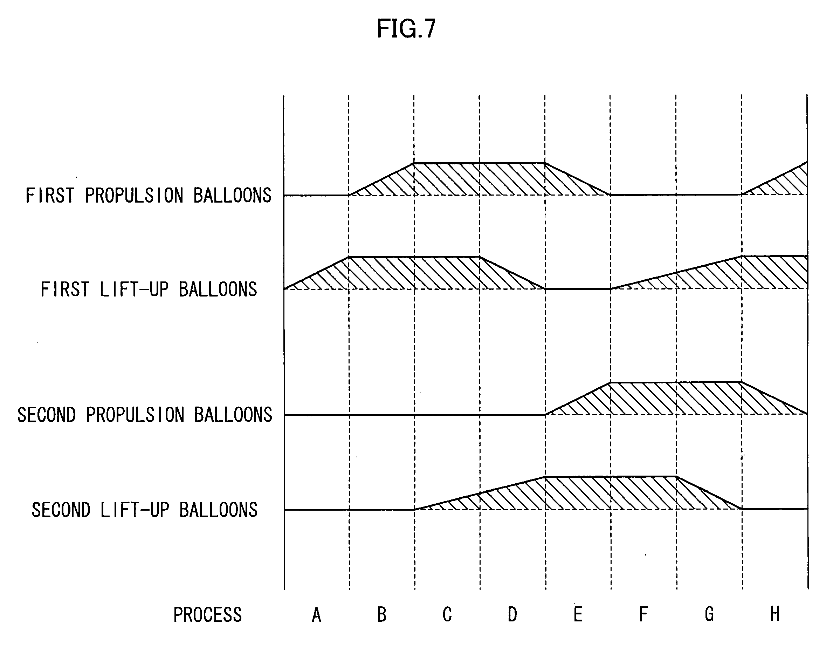

Figs. 5A and 5B are a flowchart of a specific propulsion operation of the intraductal moving body actuator of the present invention. Figs. 6A to 6J are schematic views of expansion and contraction of each balloon associated with the flowchart of the propulsion operation in Figs. 5A and 5B, left views in Figs. 6A to 6J are schematic views seen from a lateral side of the distal end portion 10a, and right views in Figs. 6A to 6J are views seen from a distal end side of the distal end portion 10a. Fig. 7 is a timing chart illustrating changes with time of pressure in each balloon in the propulsion operation in Figs. 5A and 5B.

Then, the propulsion operation flow will be described in detail on the basis of Figs. 5A and 5B with additional reference to Figs. 6A to 6J and 7.

As illustrated in Figs. 5A and 5B, the distal end portion 10a of the electronic endoscope 1 is started to be inserted into a target to be measured (for example, a large intestine), and an instruction to start self-propelled movement by the intraductal moving body actuator is given to the electronic endoscope 1 (Step S1).

Then, as propulsion balloon preparation, a predetermined amount of gas is supplied into the first propulsion balloons 32 and the second propulsion balloons 36 (Step S2).

Then, the predetermined amount of gas is supplied into the first propulsion balloons 32 and the second propulsion balloons 36 to properly expand the balloons, it is determined whether the propulsion balloon preparation is finished (Step S3), and the gas is supplied into the first propulsion balloons 32 and the second propulsion balloons 36 until the propulsion balloon preparation is finished. At this time, expansion and contraction of each balloon is shown in Fig. 6A.

When the propulsion balloon preparation is finished, filling of gas into the first lift-up balloons 30 is then started (Step S4).

Then, it is determined whether internal pressure of the first lift-up balloons 30 reaches a specified value (Step S5), the gas is filled into the first lift-up balloons 30 until the specified value is reached, and when the internal pressure of the first lift-up balloons 30 reaches the specified value, the filling of the gas into the first lift-up balloons 30 is stopped (Step S6). The specified value of the internal pressure of the first lift-up balloons 30 is a pressure value when slack in an intestinal wall 52 is removed to bring the first propulsion balloons 32 into tight contact with the intestinal wall 52, and is a pressure value at which the first propulsion balloons 32 do not break the intestinal wall 52 and do not slide on the intestinal wall 52.

At this time, expansion and contraction of each balloon is shown in Fig. 6B. As illustrated in Fig. 6B, the gas is filled into the first lift-up balloons 30 to lift up the first propulsion balloons 32, and the first propulsion balloons 32 abut against and comes into tight contact with the intestinal wall 52. A circumferential length of the intestinal wall 52 does not change, and the intestinal wall 52 is completely stretched in a direction of the first propulsion balloons 32 being lifted up. Thus, the first propulsion balloons 32 come into tight contact with the intestinal wall 52 with the slack in the intestinal wall 52 being removed. The second propulsion balloons 36 are separated from the intestinal wall 52.

At this time (Steps S4 to S6), a timing chart of each balloon corresponds to a process A in Fig. 7.

Then, filling of gas into the first propulsion balloons 32 is started (Step S7).

Then, it is determined whether internal pressure of the first propulsion balloons 32 reaches a specified value (Step S8), the gas is filled into the first propulsion balloons 32 until the internal pressure reaches the specified value, and when the internal pressure of the first propulsion balloons 32 reaches the specified value, the filling of the gas into the first propulsion balloons 32 is stopped (Step S9).

At this time, expansion and contraction of each balloon is shown in Fig. 6C. As illustrated in Fig. 6C, the gas is filled into the first propulsion balloons 32, and thus each of the first propulsion balloons 32 is directionally deformed so as to expand substantially in a fan shape rearward (the black arrow in Fig. 6C) in the advancing direction of the distal end portion 10a as shown by the dotted line in Fig. 3. Thus, a force in the advancing direction is generated on the distal end portion 10a with respect to the intestinal wall 52 as the white arrow in Fig. 6C.

In the aforementioned Step S6, the first propulsion balloons 32 reliably abut against the intestinal wall 52. Thus, the gas is filled into the first propulsion balloons 32 to reliably generate the force in the advancing direction on the distal end portion 10a with respect to the intestinal wall 52. This moves the distal end portion 10a of the electronic endoscope 1 in the advancing direction.

At this time (Steps S7 to S9), a timing chart of each balloon corresponds to a process B in Fig. 7.

Then, filling of gas into the second lift-up balloons 34 is started (Step S10).

Then, it is determined whether internal pressure of the second lift-up balloons 34 reaches a predetermined passing value (Step S11).

The predetermined passing value of the internal pressure of the second lift-up balloons 34 is a passing value present before the second lift-up balloons 34 expand and the internal pressure reaches a specified value, and is a value of the internal pressure of the second lift-up balloons 34 when the second propulsion balloons 36 abut against the intestinal wall 52.

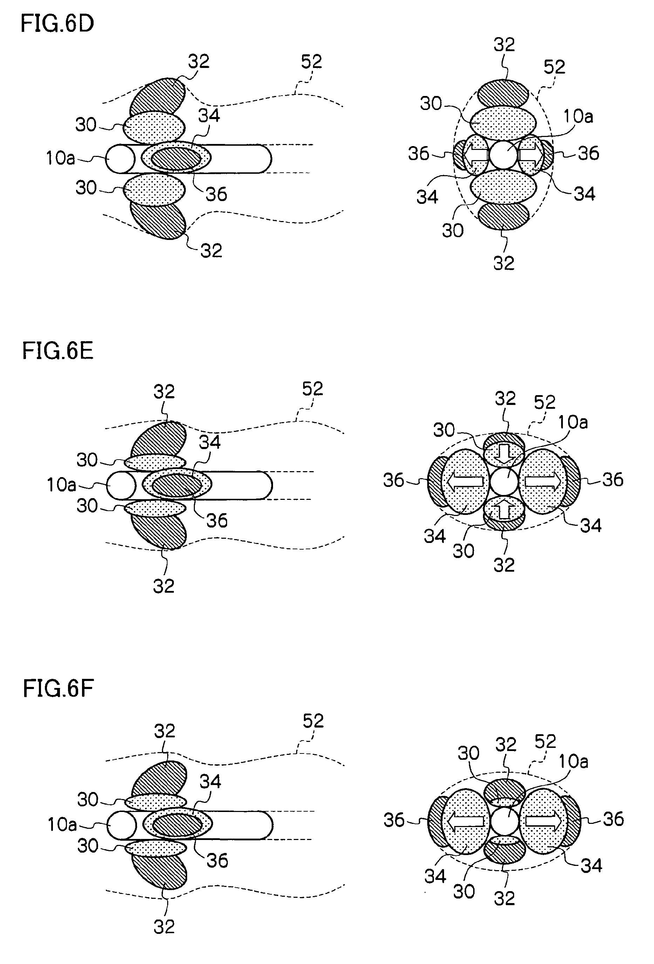

At this time, expansion and contraction of each balloon is shown in Fig. 6D. As illustrated in Fig. 6D, the gas is filled into the second lift-up balloons 34 to expand the second lift-up balloons 34 and thus lift up the second propulsion balloons 36 in a direction of the white arrow, while the first lift-up balloons 30 and the first propulsion balloons 32 are kept filled with the gas.

At this time (Steps S 10 and S11), a timing chart of each balloon corresponds to process C in Fig. 7.

Then, when the internal pressure of the second lift-up balloons 34 reaches the predetermined passing value, the second propulsion balloons 36 abut against the intestinal wall 52, and then release of the gas from the first lift-up balloons 30 is started (Step S 12).

As such, the first lift-up balloons 30 are contracted while the first propulsion balloons 32 are maintained in the expanded state. Thus, the first propulsion balloons 32 maintained in the expanded state is separated from the intestinal wall 52. Thus, an unnecessary retraction operation is not transferred to the intestinal wall 52 unlike when the first propulsion balloons 32 maintained in the contracted state are separated from the intestinal wall 52, and in Step S9, the position of movement of the distal end portion 10a of the electronic endoscope 1 in the advancing direction is maintained.

Also, the first propulsion balloons 32 are separated from the intestinal wall 52 after the second propulsion balloons 36 abut against the intestinal wall 52, and thus even if the first propulsion balloons 32 are separated from the intestinal wall 52, the distal end portion 10a is locked on the intestinal wall 52 by the second propulsion balloons 36 to maintain its position.

Next, it is determined whether the internal pressure of the second lift-up balloons 34 passes the predetermined passing value and then reaches the specified value, and whether the release of the gas from the first lift-up balloons 30 is finished (Step S 13), the gas is filled into the second lift-up balloons 34 until the internal pressure of the second lift-up balloons 34 passes the predetermined passing value and then reaches the specified value, and the gas is released from the first lift-up balloons 30 until the release of the gas from the first lift-up balloons 30 is finished. The specified value of the internal pressure of the second lift-up balloons 34 is a pressure value when slack in the intestinal wall 52 is removed to bring the second propulsion balloons 36 into tight contact with the intestinal wall 52, and is a pressure value at which the second propulsion balloons 36 do not break the intestinal wall 52 and do not slide on the intestinal wall 52.

At this time, expansion and contraction of each balloon is shown in Figs. 6E and 6F. As illustrated in Fig. 6E, the first lift-up balloons 30 are contracted while the first propulsion balloons 32 are maintained in the expanded state. Then, as illustrated in Fig. 6F, the first propulsion balloons 32 maintained in the expanded state separate from the intestinal wall 52. The filling of the gas into the second lift-up balloons 34 is continued to expand the second lift-up balloons 34.

The gas is filled into the second lift-up balloons 34 to lift up the second propulsion balloons 36, and the second propulsion balloons 36 abut against and come into tight contact with the intestinal wall 52. A circumferential length of the intestinal wall 52 does not change, and the intestinal wall 52 is completely stretched in a direction of the second propulsion balloons 36 being lifted up. Thus, the second propulsion balloons 36 come into tight contact with the intestinal wall 52 with the slack in the intestinal wall 52 being removed.

At this time (Steps S 12 and S 13), a timing chart of each balloon corresponds to a process D in Fig. 7.

When the internal pressure of the second lift-up balloons 34 reaches the specified value and the release of the gas from the first lift-up balloons 30 is finished, the filling of the gas into the second lift-up balloons 34 is stopped, the release of the gas from the first lift-up balloons 30 is stopped, filling of gas into the second propulsion balloons 36 is started, and release of the gas from the first propulsion balloons 32 is started (Step S 14).

Next, it is determined whether the internal pressure of the second propulsion balloons 36 reaches a specified value, and whether the release of the gas from the first propulsion balloons 32 is finished (Step S15), the gas is filled into the second propulsion balloons 36 until the internal pressure of the second propulsion balloons 36 reaches the specified value, and the gas is released from the first propulsion balloons 32 until the release of the gas from the first propulsion balloons 32 is finished.

When the internal pressure of the second propulsion balloons 36 reaches the specified value, the filling of the gas into the second propulsion balloons 36 is stopped, and when the release of the gas from the first propulsion balloons 32 is finished, the release of the gas from the first propulsion balloons 32 is stopped (Step S16).

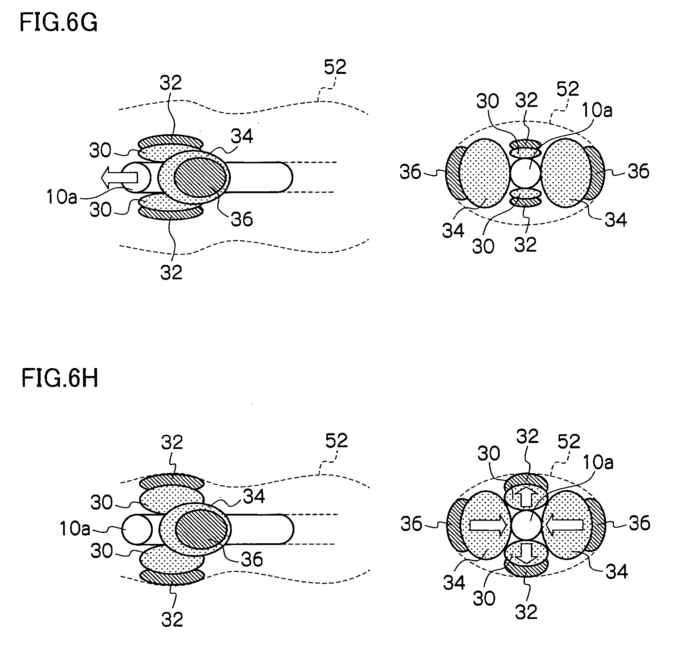

At this time, expansion and contraction of each balloon is shown in Fig. 6G. As illustrated in Fig. 6G, the first propulsion balloons 32 are contracted, while the gas is filled into the second propulsion balloons 36, and thus the second propulsion balloons 36 are directionally deformed so as to expand substantially in a fan shape rearward in the advancing direction of the distal end portion 10a as shown by the dotted line in Fig. 3. Thus, a force in the advancing direction is generated on the distal end portion 10a with respect to the intestinal wall 52 like the operation by the first propulsion balloons 32 in Fig. 6C. This moves the distal end portion 10a of the electronic endoscope 1 in the advancing direction.

At this time (Steps S 14 to S 16), a timing chart of each balloon corresponds to a process E in Fig. 7.

The filling of the gas into the second propulsion balloons 36 is stopped and the release of the gas from the first propulsion balloons 32 is stopped in Step S16, and then the process returns to Step S10. Thereafter, the first lift-up balloons 30 and the second lift-up balloons 34 are interchanged, and the first propulsion balloons 32 and the second propulsion balloons 36 are interchanged to perform the same flow, and the flow is repeated to continue the propulsion operation of the intraductal moving body actuator (Step S 17). Detailed descriptions thereof will be omitted because of its overlapping contents. At this time, expansion and contraction of each balloon is shown in Figs. 6H to 6J, and timing charts of each balloon correspond to processes F to H in Fig. 7.

Then, when an instruction to stop self-propelled movement by the intraductal moving body actuator is given to the electronic endoscope 1 (Step S 18), the release of the gas from the first propulsion balloons 32 and the second propulsion balloons 36 is started (Step S19), and the release of the gas from the first lift-up balloons 30 and the second lift-up balloon 34 is started (Step S20). When the release of the gas from all the balloons is finished, that effect is displayed on a display portion (not shown) (Step S21).

The above is the description on the propulsion operation flow.

As described above on the propulsion operation, the first propulsion balloons 32 (or second propulsion balloons 36) in the contracted state abut against the intestinal wall 52, then the gas is filled into the first propulsion balloons 32 (or second propulsion balloons 36) to expand the first propulsion balloons 32 (or second propulsion balloons 36) to apply the propulsion force to the intestinal wall 52, while the first propulsion balloons 32 (or second propulsion balloons 36) maintained in the expanded state is separated from the intestinal wall 52.

Thus, the state of the first propulsion balloons 32 (or second propulsion balloons 36) when abutted against the intestinal wall 52, and the state of the first propulsion balloons 32 (or second propulsion balloons 36) when separated from the intestinal wall 52 are made different to provide so-called hysteresis to the state of the first propulsion balloons 32 (or second propulsion balloons 36) when abutted against and separated from the intestinal wall 52.

Thus, an unnecessary retraction operation is not transferred to the intestinal wall 52 unlike when the first propulsion balloons 32 (or second propulsion balloons 36) in the contracted state separate from the intestinal wall 52. Thus, an amount of movement can be maintained of the distal end portion 10a of the electronic endoscope 1 advanced by the propulsion force generated when the first propulsion balloons 32 (or second propulsion balloons 36) are abutted against the intestinal wall 52, and a large amount of movement can be obtained in the advancing direction.

The first lift-up balloons 30 (or second lift-up balloon 34) are expanded to cause the first propulsion balloons 32 (or second propulsion balloons 36) to reliably abut against the intestinal wall 52, thereby allowing the propulsion force from the first propulsion balloons 32 (or second propulsion balloons 36) to be reliably transferred to the intestinal wall 52.

### <Variant>

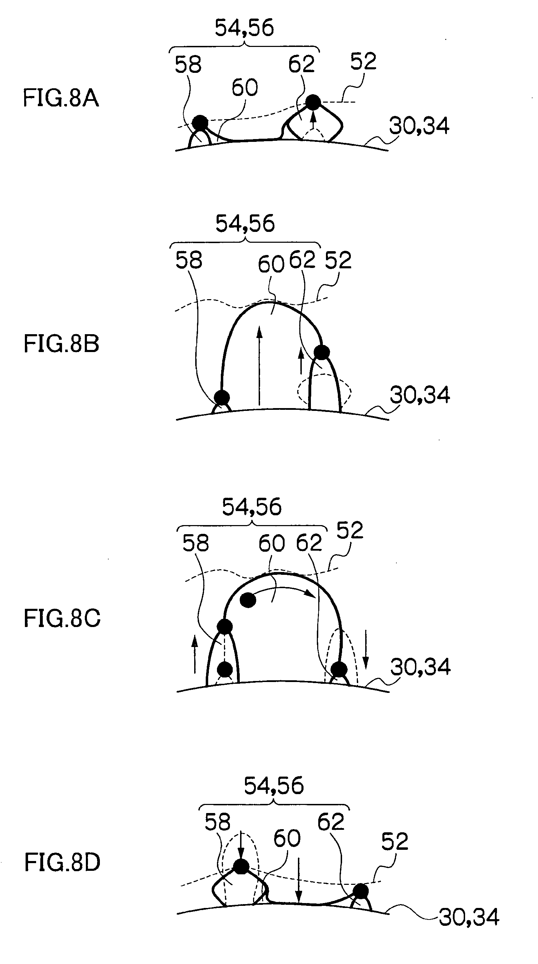

The first propulsion balloons 32 and the second propulsion balloons 36 in the above embodiment may be replaced by first propulsion balloons 54 and second propulsion balloons 56 having configurations in Fig. 8. Fig. 8 illustrates a part of a propulsion operation in use of the first propulsion balloons 54 and the second propulsion balloons 56.

The first propulsion balloons 54 and the second propulsion balloons 56 are provided to overlap upper sides (outer peripheral sides) of the first lift-up balloons 30 and the second lift-up balloons 34 so as to constitute two pairs of bilayer structure balloons α and β provided at the distal end portion 10a like the first propulsion balloons 32 and the second propulsion balloons 36 in the above embodiment.

As illustrated in Fig. 8, each first propulsion balloon 54 and each second propulsion balloon 56 are constituted by a sub-air chamber 58, a main air chamber 60, and a sub-air chamber 62 which are formed of three balloons arranged in line, and the sub-air chambers 58 and 62 are placed on opposite sides of the main air chamber 60. The sub-air chamber 58 is placed on a distal end side of the distal end portion 10a, and the sub-air chamber 62 is placed on a rear end side of the distal end portion 10a.

The first lift-up balloons 30 (or second lift-up balloons 34) cause the first propulsion balloons 54 (or second propulsion balloon 56) to abut against the intestinal wall 52, and then as illustrated in Fig. 8A, gas is filled into the sub-air chambers 62 from a state where gas is not filled into the main air chambers 60 and the sub-air chambers 58 and 62 placed on the opposite sides thereof. Then, as illustrated in Fig. 8B, gas is filled into the main air chambers 60 to bring the first propulsion balloons 54 (or second propulsion balloon 56) into tight contact with the intestinal wall 52.

Then, as illustrated in Fig. 8C, gas is filled into the sub-air chambers 58, while the gas is released from the sub-air chambers 62. Then, as shown by the arrow in FIG. 8C, a propulsion force is generated with respect to the intestinal wall 52 to move the distal end portion 10a in the advancing direction.

Then, as illustrated in Fig. 8D, the gas is released from the main air chambers 60 with the sub-air chambers 58 being expanded and the sub-air chambers 62 being contracted, and then the first lift-up balloons 30 (or second lift-up balloons 34) causes the first propulsion balloons 54 (or second propulsion balloons 56) to be separated from the intestinal wall 52.

As described above, expanded and contracted states of the sub-air chambers 58, the main air chambers 60, and the sub-air chambers 62 of the first propulsion balloons 54 (or second propulsion balloon 56) are made different between when the first propulsion balloons 54 (or second propulsion balloons 56) abut against the intestinal wall 52 and when the first propulsion balloons 54 (or second propulsion balloons 56) are separated from the intestinal wall 52. Specifically, when the first propulsion balloons 54 (or second propulsion balloon 56) abut against and separate from the intestinal wall 52, hysteresis is provided to the state of the first propulsion balloons 54 (or second propulsion balloon 56).

After the first lift-up balloons 30 (or second lift-up balloons 34) cause the first propulsion balloons 54 (or second propulsion balloons 56) to separate from the intestinal wall 52 from the state in Fig. 8C, and then as illustrated in Fig. 8D, the gas is released from the main air chambers 60 with the sub-air chambers 58 being expanded and the sub-air chambers 62 being contracted.

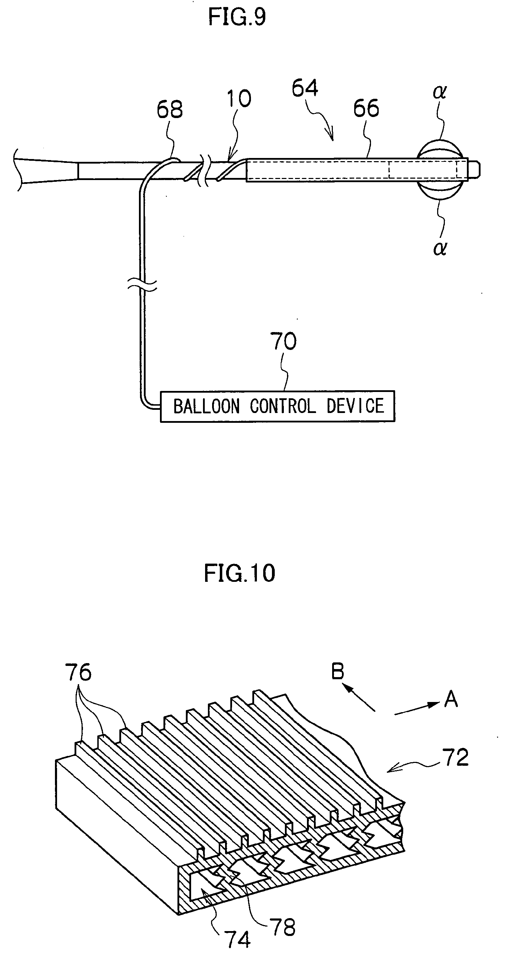

In the above embodiment, the example in which the balloons are directly mounted to the insertion portion 10 of the electronic endoscope 1 is described. The present invention is not limited thereto, but may be applied to an endoscope moving device 64 in Fig. 9.

The endoscope moving device 64 includes a cylinder 66 in which the insertion portion 10 is inserted and secured, bilayer structure balloons α and β (in Fig. 9, bilayer structure balloons α only are shown) mounted to a distal end of the cylinder 66, and a balloon control device 70 having the same configuration as the balloon control device 18 and to which a cord 68 extending from the cylinder 66 is connected. As in the above embodiment, a pair of bilayer structure balloons α are arranged axially symmetrically, and a pair of bilayer structure balloons β are arranged axially symmetrically in circumferentially 90° shifted positions. Thus, two pairs of bilayer structure balloons α and β are provided, in total. A total of three or more pairs of bilayer structure balloons may be provided.

When the insertion portion 10 is inserted into a subject, the cylinder 66 is inserted into and secured in the insertion portion 10, and the balloon control device 70 performs the same control as in the above embodiment to move the insertion portion 10.

In the above embodiment, the balloons are used as a propulsion mechanism, but, as an alternative example, elastic members 72 made of rubber or the like as illustrated in Fig. 10 may be used as a propulsion mechanism. As illustrated in Fig. 10, each elastic member 72 has a plurality of pressure chambers 74 formed in line therein in a direction A and extending substantially in parallel in a direction B perpendicular to the direction A. Protrusions 76 are formed on an upper surface at regular intervals in the direction A and extend substantially in parallel in the direction B. Diaphragms 78 between the pressure chambers 74 are formed into a bellows shape. The elastic member 72 increases and reduces pressure in the pressure chambers 74 to vertically extend and contract the diaphragms 78 and thus generate elastic deformation waves. The protrusions 76 increase amplitude of the elastic deformation waves.

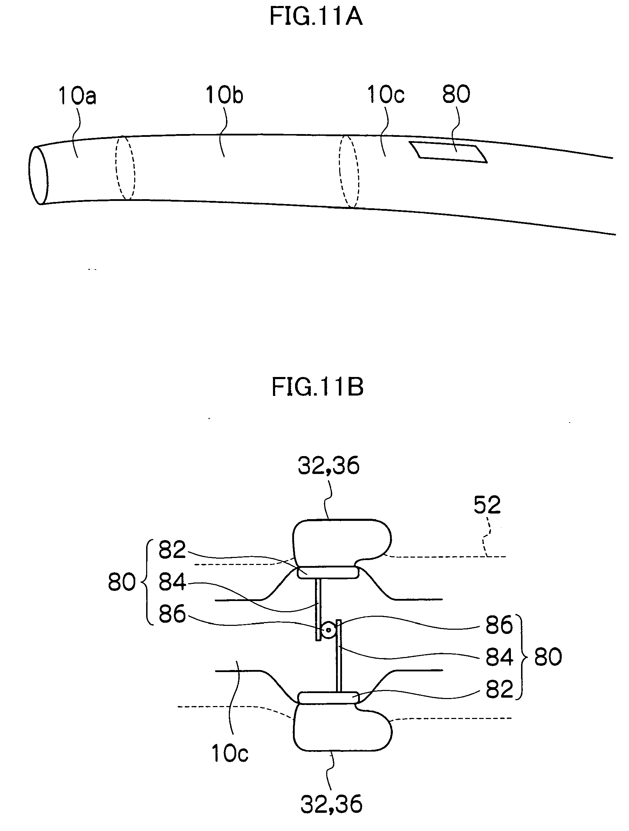

In the above embodiment, the example in which the balloons are used as a base mechanism at the distal end portion 10a (see Fig. 1) is described. Alternatively, for example, as illustrated in Figs. 11A and 11B, a lift mechanism 80 as a base mechanism may be provided on a flexible portion 10c, which has relatively more space for arranging components. For convenience of description, first propulsion balloons 32 or second propulsion balloons 36 as a propulsion mechanism is omitted in Fig. 11A.

As illustrated in Fig. 11B, a pair of lift mechanisms 80 are provided axially symmetrically on the flexible portion 10c, and each include a pad 82 as a sheet-like rigid body, a first gear 84 connected to the pad 82, and a second gear 86 that meshes with the first gear 84 and including a drive motor (not shown). The second gear 86 is shared by the pair of lift mechanisms 80. Though not shown in Figs. 11A and 11B, another pair of lift mechanisms 80 are provided in circumferentially 90° shifted positions in an axially shifted position on the flexible portion 10c.

Each of the lift mechanism 80 rotates the second gear 86 with a motor to vertically move the first gear 84 to vertically move the pad 82, and causes the first propulsion balloons 32 or the second propulsion balloons 36 as the propulsion mechanism to abut against and separate from the intestinal wall 52.

### (Example 2)

### <Configuration and operation of intraductal moving body actuator>

Figs. 2A and 2B illustrate a configuration of the intraductal moving body actuator at the distal end portion 10a of the insertion portion 10. Fig. 2A is a schematic view seen from a lateral side of the distal end portion 10a, and Fig. 2B is a view seen from a distal end side of the distal end portion 10a.

As illustrated in Figs. 2A and 2B, the intraductal moving body actuator includes first lift-up balloons 30 that are provided on an outer peripheral surface of the distal end portion 10a of the electronic endoscope 1 and lifts up first propulsion balloons 32, and the first propulsion balloons 32 provided to overlap an upper side (outer peripheral side) of the first lift-up balloons 30.

The first lift-up balloons 30 serve as a base mechanism that supports the first propulsion balloons 32 and cause the first propulsion balloons 32 to abut against and separate from a duct wall such as an intestinal wall. The first propulsion balloons 32 serve as a propulsion mechanism that generates a drive force via the duct wall such as the intestinal wall to generate a propulsion force for movement in a duct.

An interface between the first lift-up balloon 30 and the first propulsion balloons 32 is connected by bonding or integral molding.

A pair of bilayer structure balloons α as bilayer mechanisms each including the first lift-up balloon 30 and the first propulsion balloons 32 are arranged axially symmetrically at the distal end portion 10a. In the embodiment, a pair of bilayer structure balloons β as bilayer mechanisms each including a second lift-up balloon 34 and a second propulsion balloons 36 are arranged axially symmetrically at the distal end portion 10a of the endoscope in circumferentially 90° shifted positions on the distal end portion 10a. Thus, two pairs of bilayer structure balloons α and β are provided, in total. A total of three or more pairs of bilayer structure balloons may be provided.

In the embodiment, the pair of bilayer structure balloons α and the pair of bilayer structure balloons β are provided substantially in the same axial positions on the distal end portion 10a of the electronic endoscope 1, but the pair of bilayer structure balloons α and the pair of bilayer structure balloons β may be provided in axially shifted positions.

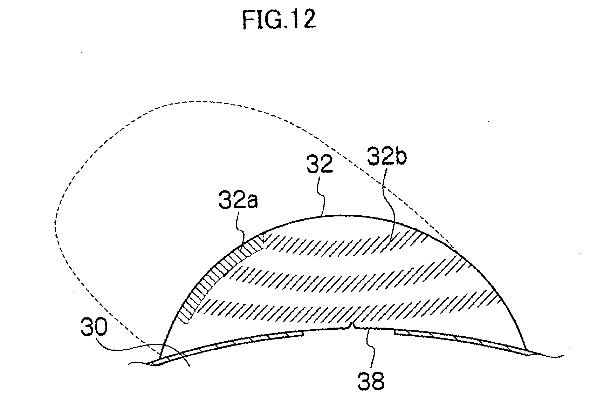

Fig. 12 is an enlarged view of the first propulsion balloons 32. Each second propulsion balloon 36 has the same configuration as the first propulsion balloon 32. The first propulsion balloons 32 are made of expandable and contractable latex rubber, for example.

As illustrated in Fig. 12, in each first propulsion balloon 32, a rear portion 32a (shaded portion) in the advancing direction of the insertion portion 10 (a direction toward the distal end portion 10a from a proximal end portion of the insertion portion 10) and a circumferential portion 32b (shaded portion) are thicker than other portions.

Thus, when gas is supplied into the first propulsion balloons 32, each of the first propulsion balloons 32 expands as shown by the dotted line in Fig. 12. Specifically, the rear portion 32a and the circumferential portion 32b are thicker than other portions, and thus the rear portion 32a and the circumferential portion 32b have a lower expansion coefficient than other portions, other portions expand more than the rear portion 32a and the circumferential portion 32b, and the first propulsion balloon 32 expands substantially in a fan shape rearward in the advancing direction as shown by the dotted line.

Fig. 12 illustrates an example in which the rear portion 32a and the circumferential portion 32b are thicker than other portions, but the rear portion 32a only may be thicker than other portions.

Low expansion coefficient material (for example, PET fiber or aramid fiber) having a lower expansion coefficient than material for the first propulsion balloons 32 or a coil-shaped wire of shape memory alloy may be joined to, embedded into, or formed integrally with surfaces of the rear portion 32a and the circumferential portion 32b or a surface of the rear portion 32a only to partially change the expansion coefficient.

As illustrated in Fig. 12, a first valve 38 is provided between the first lift-up balloon 30 and the first propulsion balloon 32. The first valve 38 has a different thickness from that of the first lift-up balloon 30 to which the first valve 38 is mounted, and the first valve 38 and the first lift-up balloon 30 have different pressure values required for expansion and contraction. In other words, the pressure required to expand and contract the first valve 38 is not the same as that to expand and contract the first lift-up balloon 30. The first valve 38 may be made of different material from that of the first lift-up balloon 30 to which the first valve 38 is mounted.

The first valve 38 is closed when internal pressure of the first lift-up balloons 30 changes from negative pressure to positive pressure or from positive pressure to negative pressure, with respect to the internal pressure of the first propulsion balloons 32, and the internal pressure of the first lift-up balloons 30 is less than a predetermined value (valve opening value), while the first valve 38 is opened when the internal pressure of the first lift-up balloons 30 reaches the predetermined value (valve opening value) or more.

The first valve 38 changes its shape between when the internal pressure of the first lift-up balloons 30 is positive pressure with respect to first propulsion balloons 32 and when the internal pressure of the first lift-up balloons 30 is negative pressure. Specifically, each first valve 38 is opened into the first propulsion balloon 32 when the internal pressure of the first lift-up balloons 30 is positive pressure at the predetermined value (valve opening value) or more, and opened into the first lift-up balloon 30 when the first lift-up balloon 30 is under negative pressure at the predetermined value (valve opening value) or more.

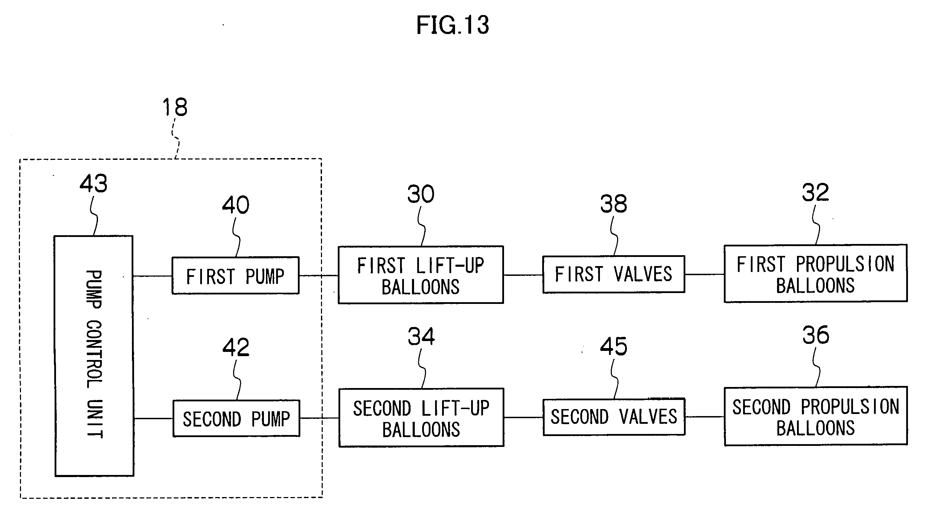

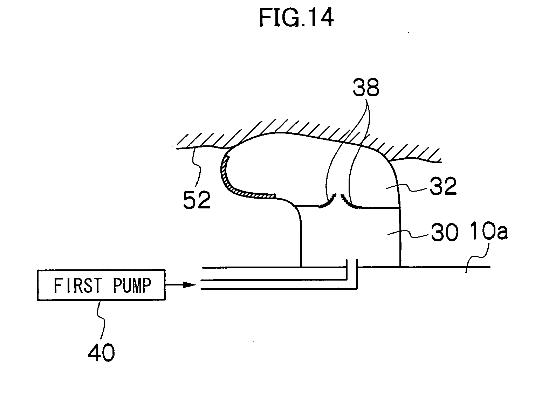

Fig. 13 is a block diagram illustrating a relationship between the balloon control device 18 for controlling pressure of the first lift-up balloons 30, the first propulsion balloons 32, the second lift-up balloons 34 and the second propulsion balloons 36, and the first valves 38 and second valves 45. Fig. 14 is a schematic view of a configuration of the first lift-up balloon 30, the first propulsion balloon 32, the first valve 38, and a first pump 40. A configuration of the second lift-up balloon 34, the second propulsion balloons 36, the second valve 45, and a second pump 42 is the same as that shown in Fig. 14.

As illustrated in Fig. 13, the balloon control device 18 includes the first pump 40, the second pump 42, and a pump control unit 43 for controlling operations of the first pump 40 and the second pump 42.

As illustrated in Figs. 13 and 14, the first lift-up balloons 30 are connected to the first pump 40 in the balloon control device 18, and each first propulsion balloon 32 is connected to the first lift-up balloon 30 via the first valve 38. Meanwhile, the second lift-up balloons 34 are connected to the second pump 42 in the balloon control device 18, and each second propulsion balloon 36 is connected to the second lift-up balloon 34 via the second valve 45.

As such, the first pump 40 is connected to both the first lift-up balloons 30 and the first propulsion balloons 32, and the second pump 42 is connected to both the second lift-up balloons 34 and the second propulsion balloons 36. Thus, one pump is enough for two types of balloons, thereby reducing cost and size of the device.

A propulsion operation flow described below is performed by the pump control unit 43 controlling the operations of the first pump 40 and the second pump 42, the first valve 38 adjusting pressure between the first lift-up balloons 30 and the first propulsion balloons 32, and the second valve 45 adjusting pressure between the second lift-up balloons 34 and the second propulsion balloons 36.

### <Propulsion operation flow>

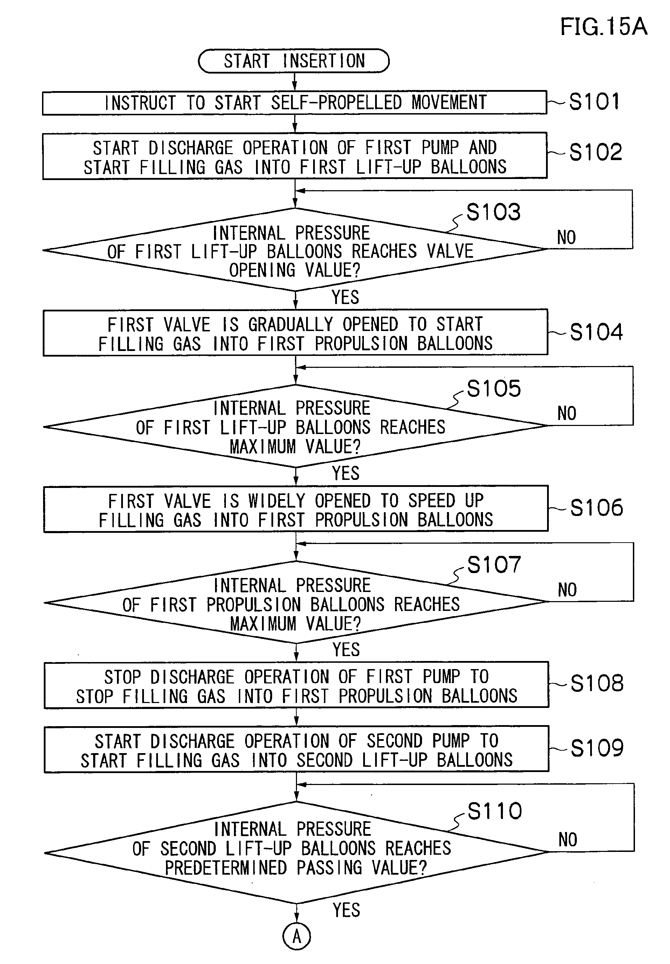

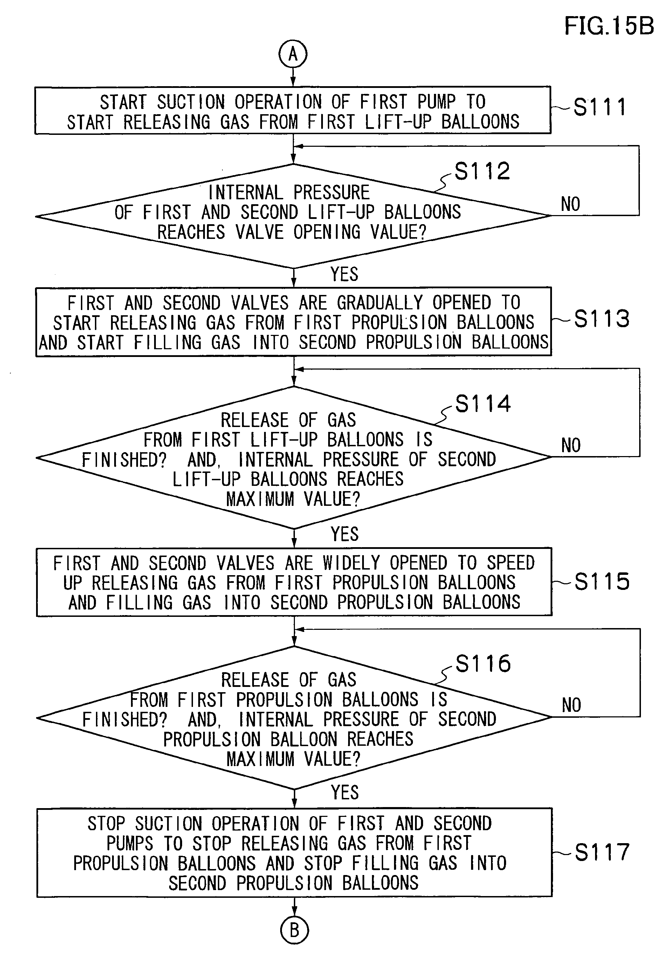

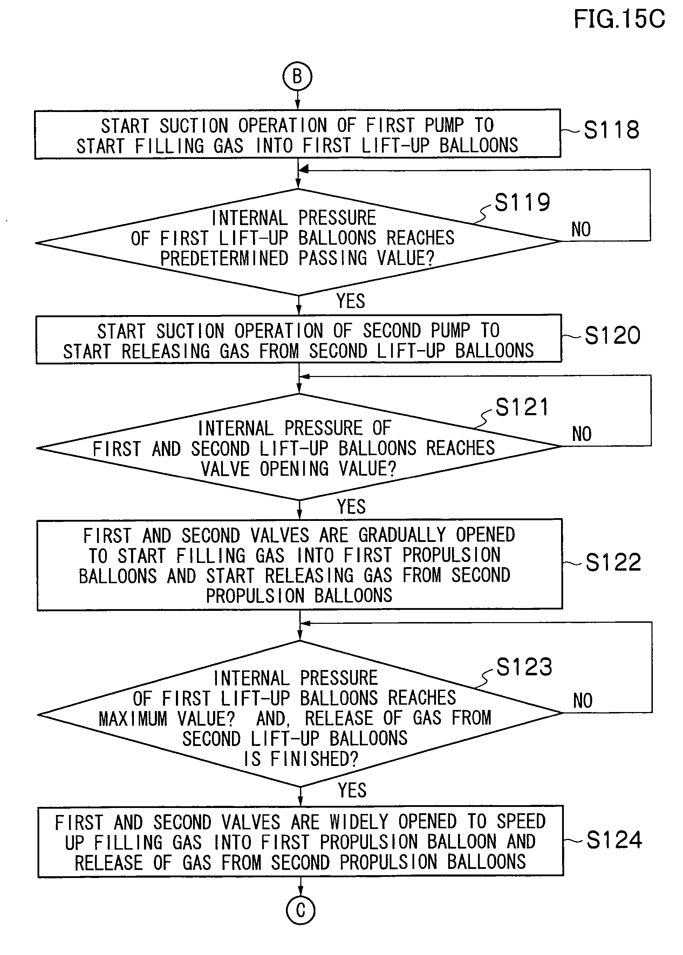

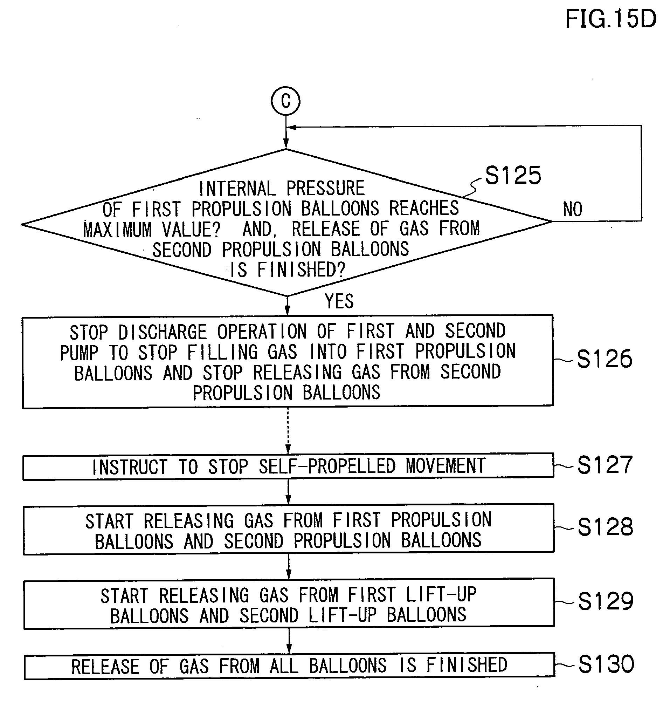

Figs. 15A to 15D are flowcharts of a detailed propulsion operation of the intraductal moving body actuator of the present invention. For convenience of description, Figs. 15A and 15B illustrate Steps S101 to S117 and Figs. 15C and 15D illustrate Steps S118 to S130.

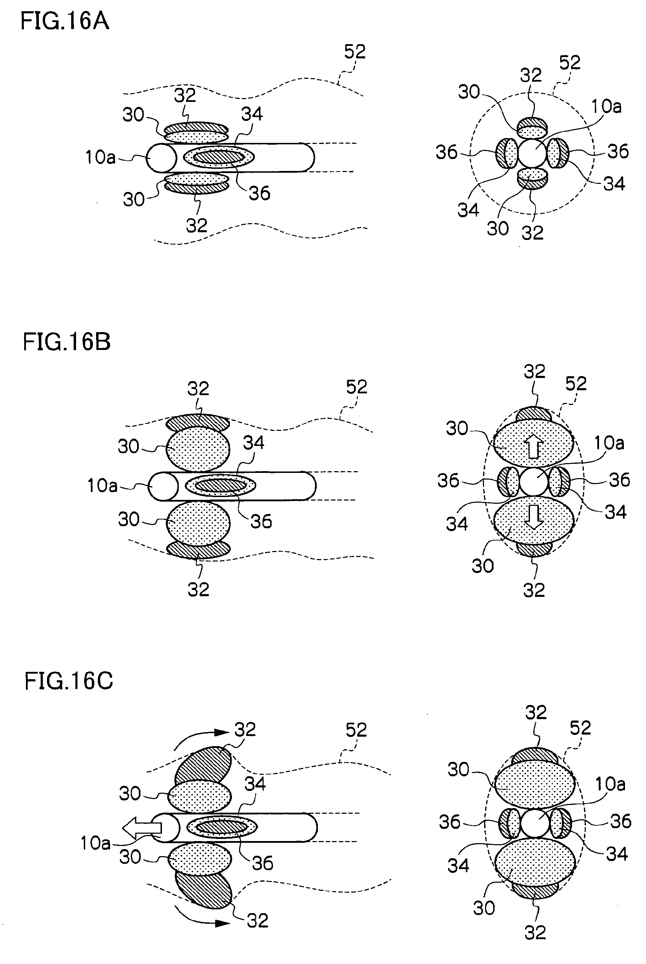

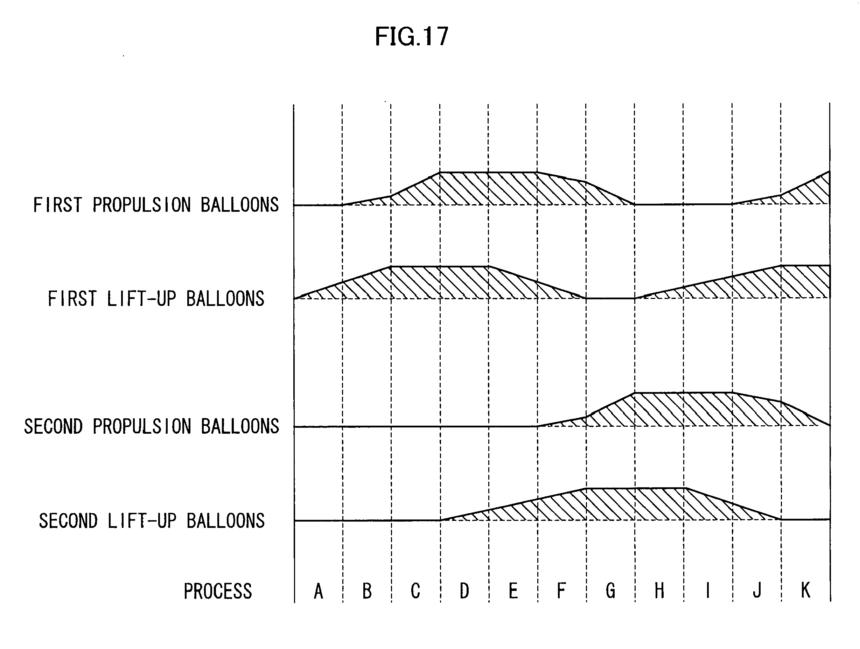

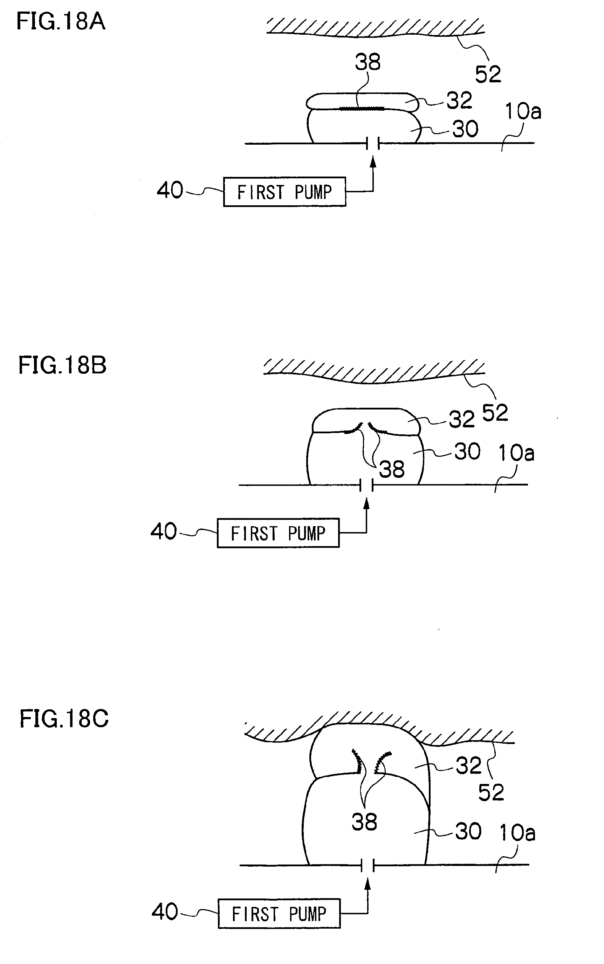

Figs. 16A to 16I are schematic views of expansion and contraction of each balloon associated with the flowchart of the propulsion operation in Figs. 15A to 15D, left views in Figs. 16A to 16I are schematic views seen from a lateral side of the distal end portion 10a, and right views in Figs. 16A to 16I are views seen from a distal end side of the distal end portion 10a. Further, Fig. 17 is a timing chart illustrating changes with time of expansion and contraction (pressure) of each balloon in the propulsion operation in Figs. 15A to 15D. Figs. 18A to 18G are schematic views of expansion and contraction of each first lift-up balloon 30 and each first propulsion balloon 32 and opening/closing of each first valve 38. Expansion and contraction of each second lift-up balloon 34 and each second propulsion balloon 36 and opening/closing of each second valve 45 are the same as those shown in Figs. 18A to 18G.

Then, the propulsion operation flow will be described in detail on the basis of Figs. 15A to 15D with additional reference to Figs. 16A to 16I and 17.

As illustrated in Figs. 15A to 15D, the distal end portion 10a of the electronic endoscope 1 is started to be inserted into a target to be measured (for example, a large intestine), and an instruction to start self-propelled movement by the intraductal moving body actuator is given to the electronic endoscope 1 (Step S101).

Then, a discharge operation of the first pump 40 is started from a state of expansion and contraction of each balloon shown in Fig. 16A to start filling gas into the first lift-up balloons 30 (Step S102). At this time, the internal pressure of each first lift-up balloons 30 is positive pressure with respect to that of each first propulsion balloons 32, but each first valve 38 is maintained in a closed state.

A time chart of expansion and contraction of each balloon in Step S 102 is shown as a process A in Fig. 17. Expansion and contraction of the first lift-up balloon 30 and the first propulsion balloons 32 and opening/closing of the first valve 38 in Step S102 are shown in Fig. 18A.

Next, when internal pressure of the first lift-up balloons 30 reaches the valve opening value, the first valves 38 are gradually opened to start filling gas into the first propulsion balloons 32 (Steps S103 and S104).

The valve opening value of the internal pressure of the first lift-up balloons 30 is an internal pressure value of the first lift-up balloons 30 when the first valves 38 are opened from the closed state.

A time chart of expansion and contraction of each balloon in Steps S 103 and S 104 is shown as a process B in Fig. 17. Expansion and contraction of each first lift-up balloon 30 and each first propulsion balloon 32 and opening/closing of each first valve 38 in Step S 104 are shown in Fig. 18B. As illustrated in Fig. 18B, the internal pressure of the first lift-up balloon 30 becomes positive pressure with respect to that of the first propulsion balloon 32, and the pressure difference between balloons 30 and 32 is equal to the valve opening value or more. Then, each first valve 38 is deformed to be opened into the first propulsion balloon 32.

Next, when the internal pressure of the first lift-up balloons 30 reaches a maximum value, the first valves 38 are widely opened to speed up filling the gas into the first propulsion balloons 32 (Steps S 105 and S 106).

The maximum value of the internal pressure of the first lift-up balloons 30 is a pressure value when slack in the intestinal wall 52 is removed to bring the first propulsion balloons 32 into tight contact with the intestinal wall 52, and is a pressure value at which the first propulsion balloons 32 do not break the intestinal wall 52 and do not slide on the intestinal wall 52.

Expansion and contraction of each balloon in Step S106 is shown in Fig. 16B. As illustrated in Fig. 16B, the gas is filled into the first lift-up balloons 30 to lift up the first propulsion balloons 32, and the first propulsion balloons 32 abut against and come into tight contact with the intestinal wall 52. A circumferential length of the intestinal wall 52 does not change, and the intestinal wall 52 is completely stretched in a direction of the first propulsion balloons 32 being lifted up. Thus, the first propulsion balloons 32 come into tight contact with the intestinal wall 52 with the slack in the intestinal wall 52 being removed. The second propulsion balloons 36 are separated from the intestinal wall 52.

Expansion and contraction of each first lift-up balloon 30 and each first propulsion balloon 32 and opening/closing of each first valve 38 in Step S106 are shown in Fig. 18C.

Next, when internal pressure of the first propulsion balloons 32 reaches a maximum value, the discharge operation of the first pump 40 is stopped to stop filling the gas into the first propulsion balloons 32 (Steps S 107 and S108).

Expansion and contraction of each balloon in Step S108 is shown in Fig. 16C. As illustrated in Fig. 16C, the gas is filled into the first propulsion balloons 32, and thus the first propulsion balloons 32 are directionally deformed so as to expand substantially in a fan shape rearward (the black arrow in Fig. 16C) in the advancing direction of the distal end portion 10a as shown by the dotted line in Fig. 12. Thus, a force in the advancing direction is generated on the distal end portion 10a with respect to the intestinal wall 52 as the white arrow in Fig. 16C.

In the Step S106, the first propulsion balloons 32 reliably abut against the intestinal wall 52. Thus, the gas is filled into the first propulsion balloons 32, and the force in the advancing direction is reliably generated on the distal end portion 10a with respect to the intestinal wall 52. This moves the distal end portion 10a of the electronic endoscope 1 in the advancing direction.

A time chart of expansion and contraction of each balloon in Steps S 105 to S 108 is shown as a process C in Fig. 17.

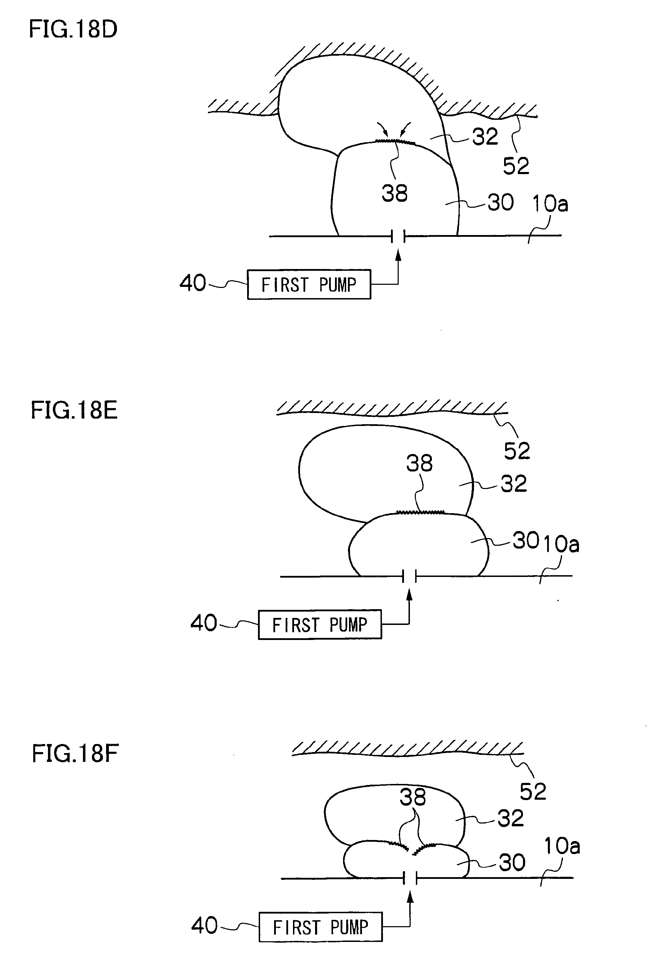

Expansion and contraction of each first lift-up balloon 30 and each first propulsion balloon 32 and opening/closing of each first valve 38 in Step S108 are shown in Fig. 18D. At this time, the first propulsion balloon 32 receives a reaction force from the intestinal wall 52, and a compressing force acts against an expanding force. However, as illustrated in Fig. 18D, the first valve 38 opened into the first propulsion balloons 32 is deformed to be closed toward the first lift-up balloon 30, and thus the internal pressure of the first propulsion balloons 32 can be maintained at a constant pressure value.

Next, a discharge operation of the second pump 42 is started to start filling gas into the second lift-up balloons 34 (Step S 109). At this time, the internal pressure of the second lift-up balloons 34 is positive pressure with respect to that of the second propulsion balloons 36, but each second valve 45 is maintained in a closed state.

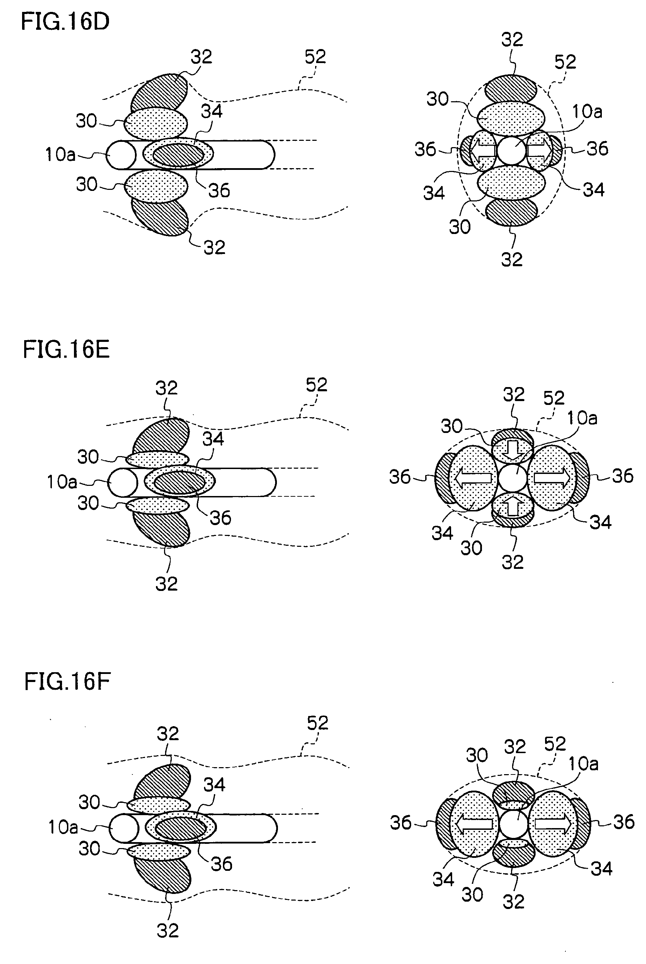

Expansion and contraction of each balloon in Step S109 is shown in Fig. 16D. As illustrated in Fig. 16D, the gas is filled into the second lift-up balloons 34 to expand the second lift-up balloons 34 and thus lift up the second propulsion balloons 36 in the direction of the white arrow, while the first lift-up balloons 30 and the first propulsion balloons 32 are kept filled with the gas.

A time chart of expansion and contraction of each balloon in Step S 109 is shown as a process D in Fig. 17.

Next, when internal pressure of the second lift-up balloons 34 reaches a predetermined passing value, a suction operation of the first pump 40 is started to start releasing the gas from the first lift-up balloons 30 (Steps S 110 and S111). At this time, the internal pressure of the first lift-up balloons 30 is changed from positive pressure to negative pressure with respect to that of the first propulsion balloons 32, but each first valve 38 is maintained in the closed state.

The predetermined passing value of the internal pressure of the second lift-up balloons 34 is a passing value which is present before the second lift-up balloons 34 expand and its internal pressure reaches a maximum value, and is a value of the internal pressure of the second lift-up balloons 34 when the second propulsion balloons 36 abut against the intestinal wall 52.

The maximum value of the internal pressure of the second lift-up balloons 34 is a pressure value when the second propulsion balloons 36 are brought into tight contact with the intestinal wall 52 with slack in the intestinal wall 52 being removed, and is a pressure value at which the second propulsion balloons 36 do not break the intestinal wall 52 and do not slide on the intestinal wall 52.

Expansion and contraction of each balloon in Step S111 is shown in Figs. 16E and 16F. As illustrated in Figs. 16E and 16F, the gas is filled into the second lift-up balloons 34 to expand the second lift-up balloons 34 and thus lift up the second propulsion balloons 36 in the direction of the white arrow and to cause the second propulsion balloons 36 to abut against the intestinal wall 52, while the release of the gas from the first lift-up balloons 30 is started to lower the first propulsion balloons 32 in the direction of the white arrow and separate the first propulsion balloons 32 from the intestinal wall 52.

A time chart of expansion and contraction of each balloon in Steps S 110 and S111 is shown as a process E in Fig. 17.

Expansion and contraction of each first lift-up balloon 30 and each first propulsion balloon 32 and opening/closing of each first valve 38 in Step S111 are shown in Fig. 18E.

As such, the first lift-up balloons 30 are contracted while the first propulsion balloons 32 are maintained in the expanded state. Thus, the first propulsion balloons 32 maintained in the expanded state separate from the intestinal wall 52. Thus, an unnecessary retraction operation is not transferred to the intestinal wall 52 unlike when the first propulsion balloons 32 maintained in the contracted state is separated from the intestinal wall 52, and in Step S108, the position of movement of the distal end portion 10a of the electronic endoscope 1 in the advancing direction is maintained.

Also, the first propulsion balloons 32 are separated from the intestinal wall 52 after the second propulsion balloons 36 abut against the intestinal wall 52, and thus even if the first propulsion balloons 32 are separated from the intestinal wall 52, the distal end portion 10a is locked on the intestinal wall 52 by the second propulsion balloons 36 to maintain its position.

Next, when the internal pressure of the first lift-up balloons 30 reaches the valve opening value, first valve 38 are gradually opened to start releasing the gas from the first propulsion balloons 32. When the internal pressure of the second lift-up balloons 34 reaches the valve opening value, the second valves 45 are gradually opened to start filling the gas into the second propulsion balloons 36 (Step S 112, S113).

A time chart of expansion and contraction of each balloon in Steps S 112 and S113 is shown as a process F in Fig. 17.

Expansion and contraction of each first lift-up balloon 30 and each first propulsion balloons 32 and opening/closing of each first valve 38 in Step S 113 are shown in Fig. 18F. As illustrated in Fig. 18F, the internal pressure of the first lift-up balloons 30 becomes negative pressure with respect to that of the first propulsion balloons 32 and the pressure difference between balloons 30 and 32 becomes equal to the valve opening value or more. Then, each first valve 38 is deformed to be opened into the first lift-up balloon 30.

Next, when the release of the gas from the first lift-up balloons 30 is finished, the first valves 38 are widely opened to speed up releasing the gas from the first propulsion balloons 32. When the internal pressure of the second lift-up balloons 34 reaches a maximum value, the second valves 45 are widely opened to stop filling the gas into the second lift-up balloons 34 to speed up filling the gas into the second propulsion balloons 36 (Steps S 114 and S 115).

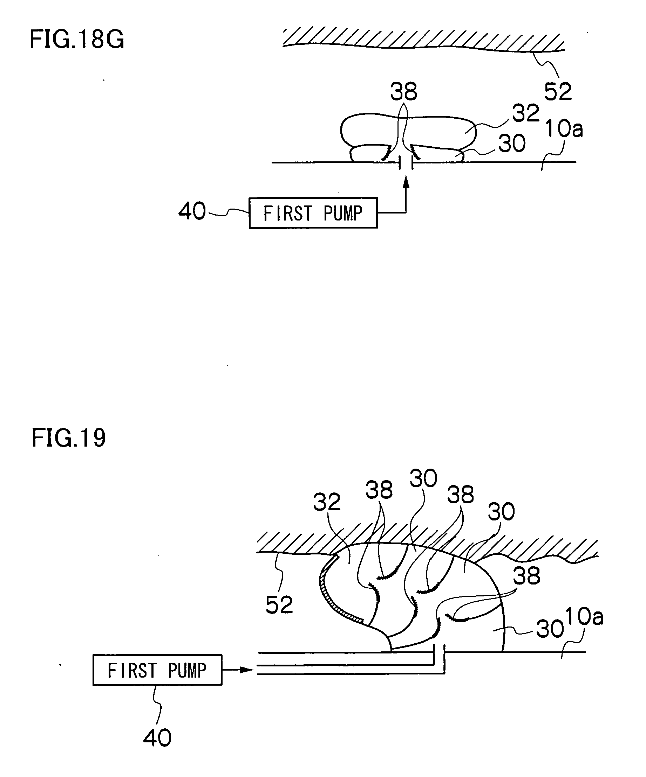

Expansion and contraction of each first lift-up balloon 30 and each first propulsion balloons 32 and opening/closing of each first valve 38 in Step S115 are shown in Fig. 18G.

Then, when the release of the gas from the first propulsion balloons 32 is finished, the suction operation of the first pump 40 is stopped, and when the internal pressure of the second propulsion balloons 36 reaches a maximum value, the discharge operation of the second pump 42 is stopped to stop filling the gas into the second propulsion balloons 36 (Steps S 116 and S 117).

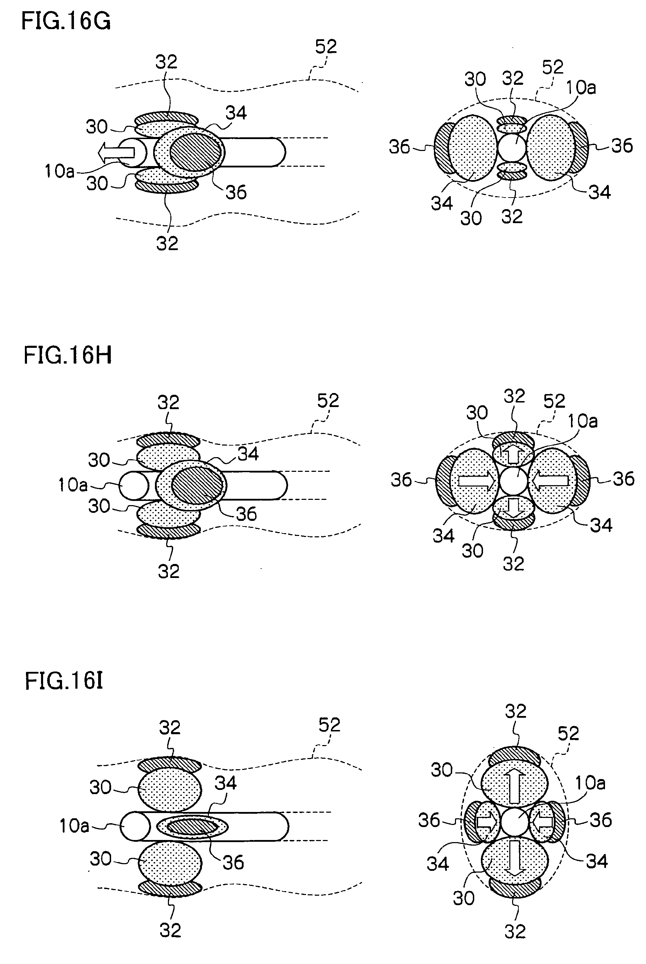

Expansion and contraction of each balloon in Step S 117 is shown in Fig. 16G. As illustrated in Fig. 16G, the first propulsion balloons 32 are contracted, while the gas is filled into the second propulsion balloons 36, and thus the second propulsion balloons 36 are directionally deformed so as to expand substantially in a fan shape rearward in the advancing direction of the distal end portion 10a as shown by the dotted line in Fig. 12. Thus, a force in the advancing direction is generated on the distal end portion 10a with respect to the intestinal wall 52 like the operation by the first propulsion balloons 32 in Fig. 16C. This moves the distal end portion 10a of the electronic endoscope 1 in the advancing direction.

A time chart of expansion and contraction of each balloon in Steps S 114 to S 117 is shown as a process G in Fig. 17.

Next, the discharge operation of the first pump 40 is started to start filling the gas into the first lift-up balloons 30 (Step S 118). At this time, the internal pressure of the first lift-up balloons 30 changes from negative pressure to positive pressure with respect to that of the first propulsion balloons 32, but the first valves 38 are maintained in the closed state.

A time chart of expansion and contraction of each balloon in Step S 118 is shown as a process H in Fig. 17.

Next, when the internal pressure of the first lift-up balloons 30 reaches a predetermined passing value, a suction operation of the second pump 42 is started to start releasing the gas from the second lift-up balloons 34 (Steps S 119 and S120). At this time, the internal pressure of the second lift-up balloons 34 changes from positive pressure to negative pressure with respect to that of the second propulsion balloons 36, but the first valves 38 are maintained in the closed state.

The predetermined passing value of the internal pressure of the first lift-up balloons 30 is a passing value which is present before the first lift-up balloons 30 expand and its internal pressure reaches a maximum value, and is a value of the internal pressure of the first lift-up balloons 30 when the first propulsion balloons 32 abut against the intestinal wall 52.

Expansion and contraction of each balloon in Step S120 is shown in Figs. 16H and 16I. As illustrated in Figs. 16H and 16I, the first lift-up balloons 30 are expanded to lift up the first propulsion balloons 32 in the direction of the white arrow and cause the first propulsion balloons 32 to abut against the intestinal wall 52, while the gas is released from the second lift-up balloons 34 to lower the second propulsion balloons 36 and separate the second propulsion balloons 36 from the intestinal wall 52.

A time chart of expansion and contraction of each balloon in Steps S 119 and S120 is shown as a process I in Fig. 17.

Next, when the internal pressure of the first lift-up balloons 30 reaches the valve opening value, the first valves 38 are gradually opened to start filling the gas into the first propulsion balloons 32. When the internal pressure of the second lift-up balloons 34 reaches the valve opening value, the second valves 45 are gradually opened to start releasing the gas from the second propulsion balloons 36 (Steps S121 and S122).

A time chart of expansion and contraction of each balloon in Steps S121 and S122 is shown as a process J in Fig. 17.

Next, when the internal pressure of the first lift-up balloons 30 reaches a maximum value, the first valves 38 are widely opened to stop filling the gas into the first lift-up balloons 30 and also speed up filling the gas into the first propulsion balloons 32. When the release of the gas from the second lift-up balloons 34 is finished, the second valves 45 are widely opened to speed up the release of the gas from the second propulsion balloons 36 (Steps S123 and S124).

Then, when the internal pressure of the first propulsion balloons 32 reaches a maximum value, the discharge operation of the first pump 40 is stopped to stop filling the gas into the first propulsion balloons 32. When the release of the gas from the second propulsion balloons 36 is finished, the discharge operation of the second pump 42 is stopped to stop the release of the gas from the second propulsion balloons 36 (Steps S125 and S126).

Expansion and contraction of each balloon in Step S126 is shown in Fig. 16C, and a force in the advancing direction is generated on the distal end portion 10a with respect to the intestinal wall 52 as the white arrow in Fig. 16C.

In Step S120, the first propulsion balloons 32 reliably abut against the intestinal wall 52, and thus the gas is filled into the first propulsion balloons 32 to reliably generate the force in the advancing direction on the distal end portion 10a with respect to the intestinal wall 52. This moves the distal end portion 10a of the electronic endoscope 1 in the advancing direction.

A time chart of expansion and contraction of each balloon in Steps S 123 to S126 is shown as a process K in Fig. 17.

Next, the discharge operation of the second pump 42 is started to start filling the gas into the second lift-up balloons 34, and thereafter Steps S 109 to S126 are repeated, and thus the descriptions thereof will be omitted.

Then, when an instruction to stop self-propelled movement by the intraductal moving body actuator is given to the electronic endoscope 1 (Step S 127), the release of the gas from the first propulsion balloons 32 and the second propulsion balloons 36 is started (Step S128), and the release of the gas from the first lift-up balloons 30 and the second lift-up balloons 34 is started (Step S129). When the release of the gas from all the balloons is finished, that effect is displayed on a display portion (not shown) (Step S130).

The above is the description on the propulsion operation flow.

As described above on the propulsion operation, the first valve 38 is provided between each first lift-up balloon 30 and each first propulsion balloon 32, the second valve 45 is provided between each second lift-up balloon 34 and each second propulsion balloon 36, and the pump control unit 43 in the balloon control device 18 drives the operations of the first pump 40 and the second pump 42, thereby reducing the number of pumps to control pressure in each balloon and reducing cost and size of the device.

The first propulsion balloons 32 (or second propulsion balloons 36) in the contracted state abut against the intestinal wall 52, then the gas is filled into the first propulsion balloons 32 (or second propulsion balloons 36) to expand the first propulsion balloons 32 (or second propulsion balloons 36) to apply the propulsion force to the intestinal wall 52, while the first propulsion balloons 32 (or second propulsion balloons 36) maintained in the expanded state are separated from the intestinal wall 52.

Thus, the state of the first propulsion balloons 32(or second propulsion balloons 36) when they abut against the intestinal wall 52, and the state of the first propulsion balloons 32 (or second propulsion balloons 36) when they separate from the intestinal wall 52 are made different to provide so-called hysteresis to the state of the first propulsion balloons 32 (or second propulsion balloons 36) when they abut against and separate from the intestinal wall 52.

Thus, an unnecessary retraction operation is not transferred to the intestinal wall 52 unlike when the first propulsion balloons 32 (or second propulsion balloons 36) in the contracted state are separated from the intestinal wall 52. Thus, an amount of movement can be maintained of the distal end portion 10a of the electronic endoscope 1 advanced by the propulsion force generated when the first propulsion balloons 32 (or second propulsion balloons 36) abut against the intestinal wall 52, and a large amount of movement can be obtained in the advancing direction.

The first lift-up balloons 30 (or second lift-up balloon 34) are expanded to cause the first propulsion balloons 32 (or second propulsion balloons 36) to reliably abut against the intestinal wall 52, thereby allowing the propulsion force from the first propulsion balloons 32 (or second propulsion balloons 36) to be reliably transferred to the intestinal wall 52.

In the above embodiment, the bilayer structure balloons are used, but not limited thereto, balloons in three or more layers may be used in which one or more first lift-up balloons 30 (or second lift-up balloons 34) and first valves 38 (or second valves 45) are provided, and one or more first propulsion balloons 32 (or second propulsion balloons 36) are provided thereon.

Fig. 19 illustrates, as an example, a four-layer structure balloon in which first lift-up balloons 30 (or second lift-up balloons 34) and first valves 38 (or second valves 45) are provided in three layers, and a first propulsion balloons 32 (or second propulsion balloons 36) is provided thereon.

### <Variant>

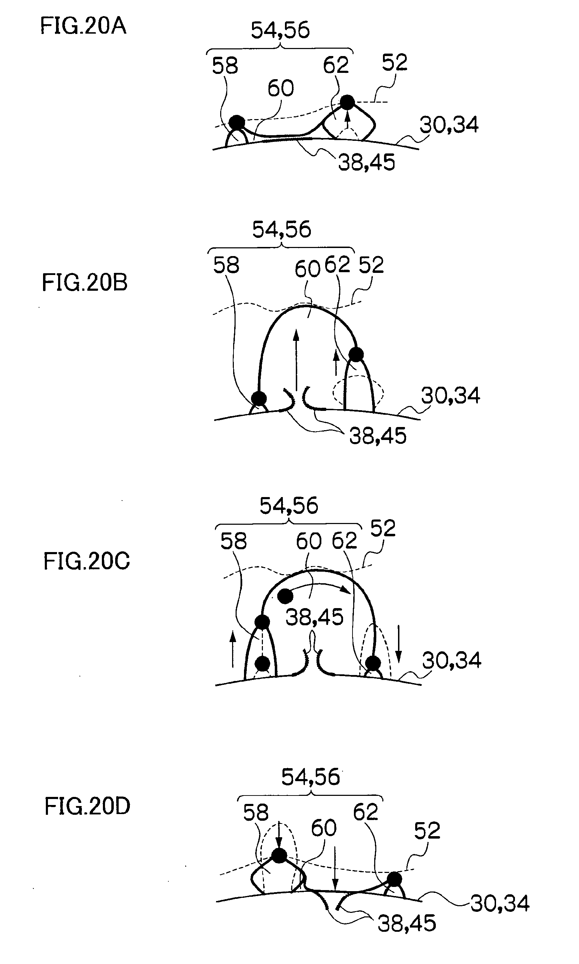

The first propulsion balloons 32 and the second propulsion balloons 36 in the above embodiment may be replaced by a first propulsion balloons 54 and a second propulsion balloons 56 having configurations in Fig. 20. Fig. 20 illustrates a part of a propulsion operation in use of the first propulsion balloons 54 and the second propulsion balloons 56.

The first propulsion balloon 54 and the second propulsion balloon 56 are provided to overlap upper sides (outer peripheral sides) of each first lift-up balloon 30 and each second lift-up balloon 34 so as to constitute two pairs of bilayer structure balloons α and β provided at the distal end portion 10a like the first propulsion balloon 32 and the second propulsion balloon 36 in the above embodiment.

As illustrated in Fig. 20, each first propulsion balloon 54 and each second propulsion balloon 56 includes a sub-air chamber 58, a main air chamber 60, and a sub-air chamber 62 formed of three balloons arranged in line, and the sub-air chambers 58 and 62 are placed on opposite sides of the main air chamber 60. The sub-air chamber 58 is placed on a distal end side of the distal end portion 10a, and the sub-air chamber 62 is placed on a rear end side of the distal end portion 10a.

The first lift-up balloon 30 (or second lift-up balloon 34) causes the first propulsion balloon 54 (or second propulsion balloon 56) to abut against the intestinal wall 52, and then as illustrated in Fig. 20A, gas is filled into the sub-air chamber 62 from a state where gas is not filled into the main air chamber 60 and the sub-air chambers 58 and 62 placed on the opposite sides thereof. Then, as illustrated in Fig. 20B, gas is filled into the main air chamber 60 to bring the first propulsion balloon 54 (or second propulsion balloon 56) into tight contact with the intestinal wall 52.

Then, as illustrated in Fig. 20C, gas is filled into the sub-air chamber 58, while the gas is released from the sub-air chamber 62. Then, as shown by the arrow in FIG. 20C, a propulsion force is generated with respect to the intestinal wall 52 to move the distal end portion 10a in the advancing direction.

Then, as illustrated in Fig. 20D, the gas is released from the main air chamber 60 with the sub-air chamber 58 being expanded and the sub-air chamber 62 being contracted, and then the first lift-up balloon 30 (or second lift-up balloon 34) causes the first propulsion balloon 54 (or second propulsion balloon 56) to be separated from the intestinal wall 52.

As described above, expanded and contracted states of the sub-air chamber 58, the main air chamber 60, and the sub-air chamber 62 of each first propulsion balloon 54 (or second propulsion balloon 56) are made different between when the first propulsion balloon 54 (or second propulsion balloon 56) abuts against the intestinal wall 52 and when the first propulsion balloon 54 (or second propulsion balloon 56) separates from the intestinal wall 52. Specifically, when the first propulsion balloon 54 (or second propulsion balloon 56) abut against and separate from the intestinal wall 52, hysteresis is provided to the state of the first propulsion balloon 54 (or second propulsion balloon 56).

It may be allowed that the first lift-up balloon 30 (or second lift-up balloon 34) causes the first propulsion balloon 54 (or second propulsion balloon 56) to separate from the intestinal wall 52 from the state in Fig. 20C, and then as illustrated in Fig. 20D, the gas is released from the main air chamber 60 with the sub-air chamber 58 being expanded and the sub-air chamber 62 being contracted.

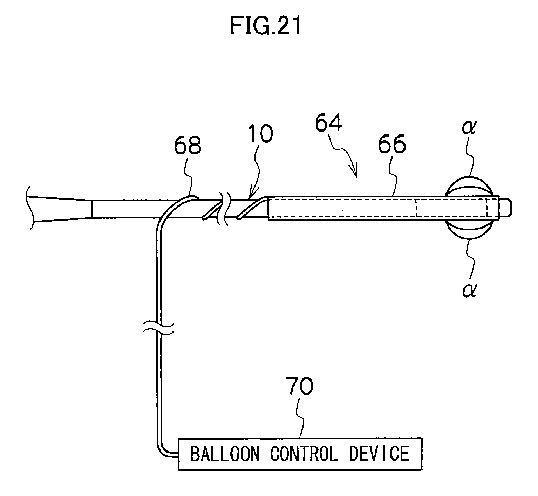

In the above embodiment, the example in which the balloons are directly mounted to the insertion portion 10 of the electronic endoscope 1 is described. The present invention is not limited thereto, but may be applied to an endoscope moving device 64 in Fig. 21.

The endoscope moving device 64 includes a cylinder 66 in which an insertion portion 10 is inserted and secured, bilayer structure balloons α and β (in Fig. 21, bilayer structure balloons α only are shown) mounted to a distal end of the cylinder 66, and a balloon control device 70 having the same configuration as the balloon control device 18 and to which a cord 68 extending from the cylinder 66 is connected. As in the above embodiment, a pair of bilayer structure balloons α are arranged axially symmetrically, and a pair of bilayer structure balloons β are arranged axially symmetrically in circumferentially 90° shifted positions. Thus, two pairs of bilayer structure balloons α and β are provided, in total. A total of three or more pairs of bilayer structure balloons may be provided.

When the insertion portion 10 is inserted into a subject, the cylinder 66 is inserted into and secured in the insertion portion 10, and the balloon control device 70 performs the same control as in the above embodiment to move the insertion portion 10.

### [Second embodiment]

### [Description on electronic endoscope]

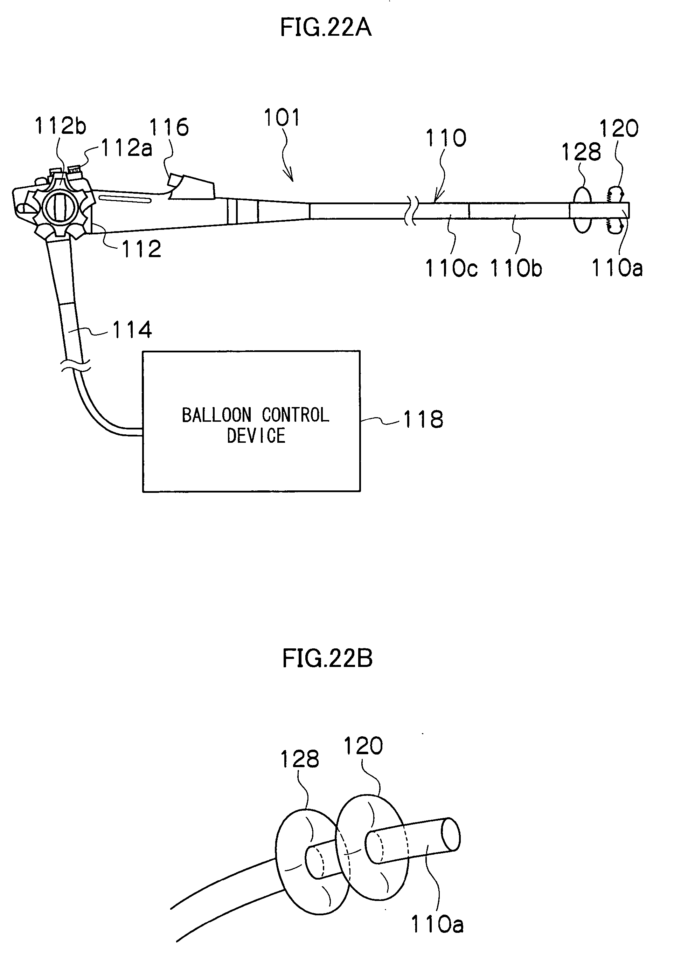

In Fig. 22A, an electronic endoscope 101 includes an insertion portion 110 to be inserted into a subject, and an operation portion 112 connected to a proximal end portion of the insertion portion 110. A distal end portion 110a (for example, having an outer diameter of 12 mmφ) connected to a distal end of the insertion portion 110 includes an objective lens for capturing an image light of an observed region in the subject, and an imaging device for picking up an image of the image light (both not shown). The image of the inside of the subject obtained by the imaging device is displayed as an endoscope image on a monitor of a processor device (both not shown) connected to a cord 114.

The distal end portion 110a also includes an illumination window for applying illumination light from a light source device (not shown) to the observed region, a forceps exit communicating with a forceps opening 116, and a nozzle for spraying cleaning water or air for cleaning an observation window for protecting the objective lens by operating an air/water supply button 112a.

A bending portion 110b to which a plurality of bending pieces are connected is provided behind the distal end portion 110a. The bending portion 110b vertically and laterally bends by an angle knob 112b provided on the operation portion 112 being operated to push and pull a wire inserted through the insertion portion 110. Thus, the distal end portion 110a is oriented in a desired direction in the subject.

A flexible portion 110c is provided behind the bending portion 110b. The flexible portion 110c has a length of one to several meters so that the distal end portion 110a can reach the observed region and an operator can keep a proper distance from a patient so that the operator may grip and operate the operation portion 12 without any trouble.

To the distal end portion 110a, propulsion balloons 120 and 142 and lock balloons 128 and 156 or bilayer structure balloons described later are mounted (in Figs. 22A and 22B, an example in which the propulsion balloon 120 and the lock balloon 128 are mounted is shown). The propulsion balloons 120 and 142 and the lock balloons 128 and 156 or the bilayer structure balloons are mainly made of, for example, expandable and contractable latex rubber, and connected to a balloon control device 18 for controlling pressure in each balloon.

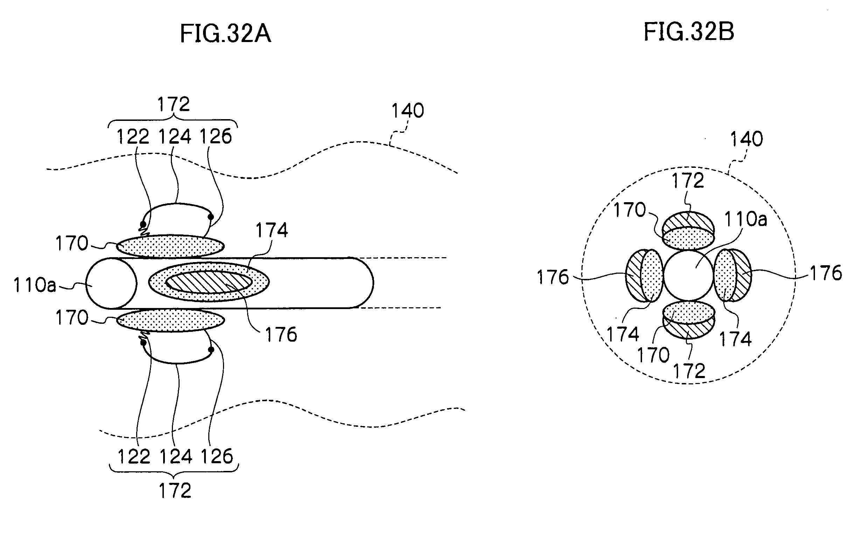

As illustrated in Fig. 22B, the propulsion balloon 120 and the lock balloon 128 are formed entirely circumferentially of the insertion portion 110. The bilayer structure balloons are arranged opposite to each other circumferentially of the insertion portion 110, that is, 180° apart (see Fig. 32).

In Figs. 22A and 22B, the propulsion balloon 120 is mounted on a front side of the distal end portion 110a and the lock balloon 128 is mounted on a rear side of the distal end portion 110a, but not limited thereto, the lock balloon 128 may be mounted on the front side of the distal end portion 110a and the propulsion balloon 120 may be mounted on the rear side of the distal end portion 110a.

When an inner wall surface of a duct complexly bent such as a large intestine or a small intestine is observed with the electronic endoscope 101 configured as described above, the insertion portion 110 is inserted into the subject with the propulsion balloons 120 and 142 and the lock balloons 128 and 156 or the bilayer structure balloons being contracted, the light source device is lit to illuminate the inside of the subject, and an endoscope image obtained by the imaging device is observed by the monitor.

When the distal end portion 10a reaches the duct, the balloon control device 118 controls expansion and contraction of the propulsion balloons 120 and 142 and the lock balloons 128 and 156 or the bilayer structure balloons to generate a drive force via the inner wall surface of the duct, and the force moves the insertion portion 110 in the advancing direction.

A propulsion operation flow will be described in detail later.

### [Description on intraductal moving body actuator]

Next, an intraductal moving body actuator will be described.

### (Example 1)

### <Configuration of intraductal moving body actuator>

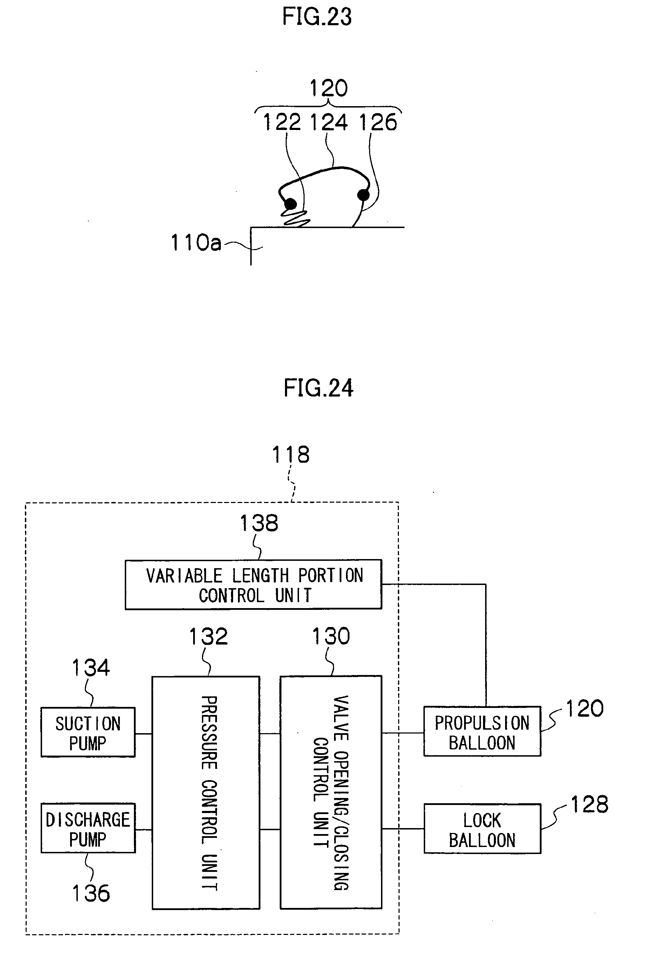

Fig. 23 is an enlarged sectional view of the propulsion balloon 120 as a propulsion mechanism in Example 1 at the distal end portion 110a of the insertion portion 110. As illustrated in Fig. 23, the propulsion balloon 120 includes a fixed length portion 124 as a first region, and a first variable length portion 122 and a second variable length portion 126 as a pair of second regions connected to opposite ends of the fixed length portion 124.

A detailed configuration of the propulsion balloon 120 includes a configuration in which the entire propulsion balloon 120 is made of expandable and contractable latex rubber, and shape memory material, artificial muscle or the like is bonded to the first variable length portion 122 and the second variable length portion 126 only, or a configuration in which the fixed length portion 124 only is made of expandable and contractable latex rubber, and the first variable length portion 122 and the second variable length portion 126 are made of shape memory material, artificial muscle or the like.

For the configuration in which the entire propulsion balloon 120 is made of expandable and contractable latex rubber, and shape memory material, artificial muscle or the like is bonded to the first variable length portion 122 and the second variable length portion 126 only, the shape memory material, the artificial muscle or the like may be bonded to the entire surface corresponding to the first variable length portion 122 and the second variable length portion 126, or to surfaces corresponding to the first variable length portion 122 and the second variable length portion 126 in streaks.

The fixed length portion 124 is a portion with a fixed length during a propulsion operation described later. The first variable length portion 122 and the second variable length portion 126 are portions of shape memory material, artificial muscle or the like with variable lengths by extension and contraction during the propulsion operation described later.

Fig. 24 is a block diagram of a balloon control device 118 for controlling pressure of the propulsion balloon 120 and the lock balloon 128. As illustrated in Fig. 24, the balloon control device 118 has a configuration in which the propulsion balloon 120 and the lock balloon 128 can be independently adjusted in internal pressure, and a suction pump 134 and a discharge pump 136 are connected to the balloons via a valve opening/closing control unit 130 and a pressure control unit 132.

The balloon control device 118 also includes a variable length portion control unit 138 for controlling the first variable length portion 122 and the second variable length portion 126 of the propulsion balloon 120.

A propulsion operation flow described below is performed by the valve opening/closing control unit 130 controlling opening/closing of a valve (not shown) connected to each balloon, the pressure control unit 132 controlling the suction pump 134 and the discharge pump 136, and the variable length portion control unit 138 heating or cooling the first variable length portion 122 and the second variable length portion 126 of the propulsion balloon 120 so that the variable length portions are contracted or extended.

### <Propulsion operation flow>

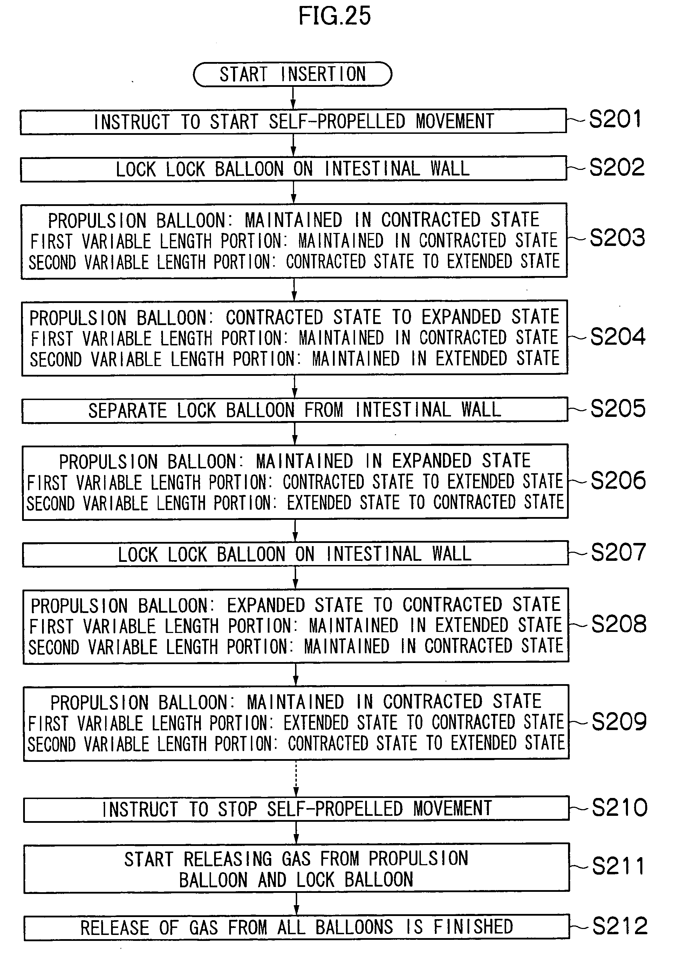

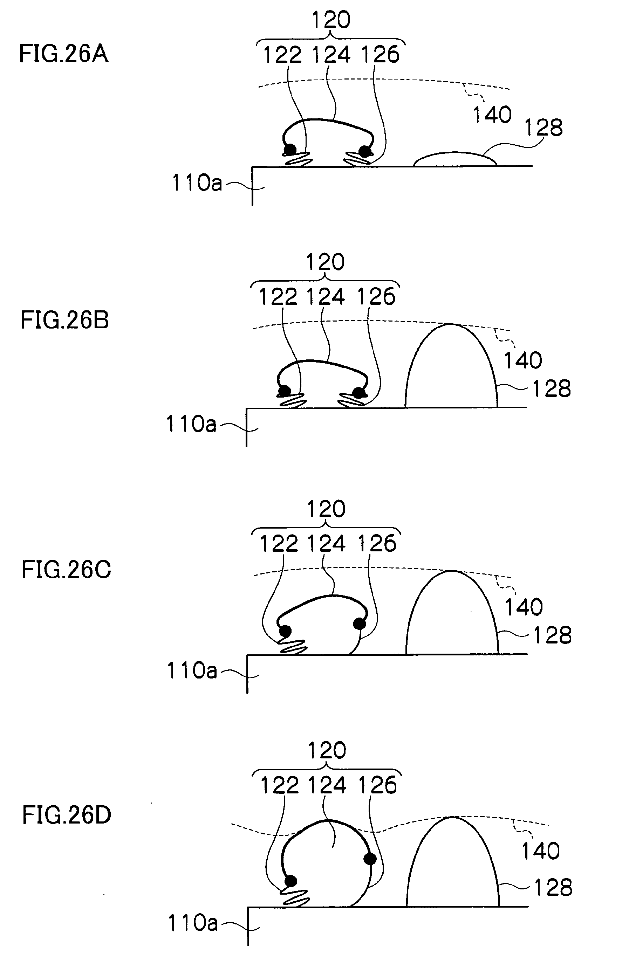

Fig. 25 is a flowchart of a detailed propulsion operation in Example 1 of the intraductal moving body actuator of the present invention. Figs. 26A to 26H are schematic sectional views of expansion and contraction of each balloon associated with the flowchart of the propulsion operation in Fig. 25.

The propulsion operation flow will be described in detail on the basis of Fig. 25 with additional reference to Figs. 26A to 26H.

First, as illustrated in Fig. 26A, the distal end portion 110a of the electronic endoscope 101 is started to be inserted into a target to be measured (for example, a large intestine) with both the propulsion balloon 120 and the lock balloon 128 being contracted, and an instruction to start self-propelled movement by the intraductal moving body actuator is given to the electronic endoscope 1 electronic endoscope 101 (Step S201).

Then, gas is filled into the lock balloon 128 to expand the lock balloon 128 and lock the lock balloon 128 on an intestinal wall 140 (Step S202). Expansion and contraction of the balloons in Step S202 is shown in Fig. 26B.

Then, with the propulsion balloon 120 being maintained in the contracted state, the first variable length portion 122 is maintained in a contracted state, and the second variable length portion 126 is extended (Step S203). Expansion and contraction of the balloons in Step S203 is shown in Fig. 26C.

Then, gas is filled into the propulsion balloon 120 until internal pressure reaches a specified value to change the propulsion balloon 120 from the contracted state to an expanded state (Step S204). Expansion and contraction of the balloons in Step S204 is shown in Fig. 26D. As illustrated in Fig. 26D, the propulsion balloon 120 (particularly, the fixed length portion 124 of the propulsion balloon 120) abuts against and comes into contact with the intestinal wall 140.

The specified value of the internal pressure of the propulsion balloon 120 is a pressure value when the propulsion balloon 120 is brought into tight contact with the intestinal wall 140 with slack in the intestinal wall 140 being removed, and is a pressure value at which the propulsion balloon 120 does not break the intestinal wall 140 and does not slide on the intestinal wall 140.

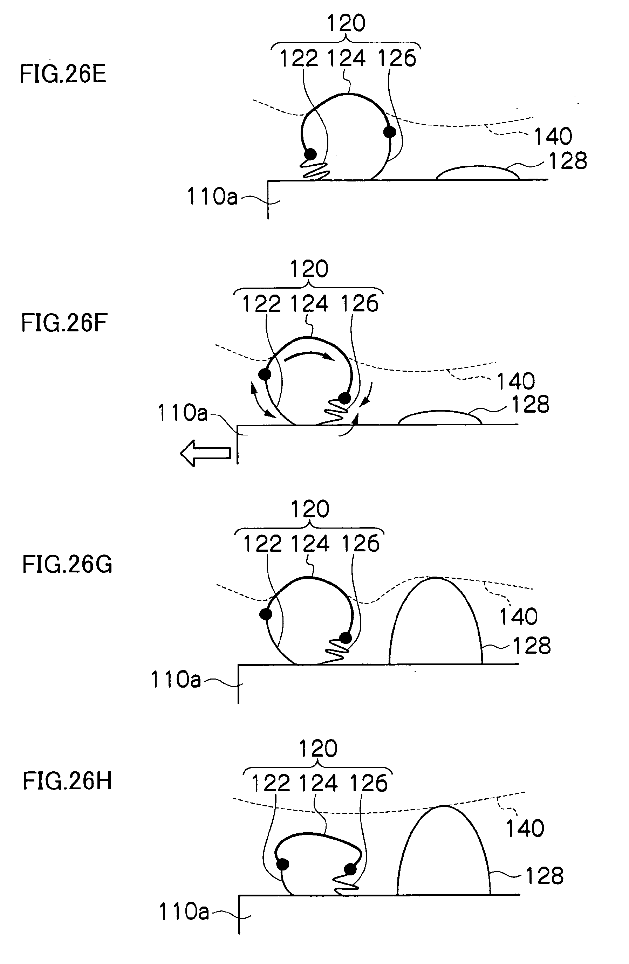

Then, the gas is released from the lock balloon 128 to separate the lock balloon 128 from the intestinal wall 140 (Step S205). Expansion and contraction of the balloons in Step S205 is shown in Fig. 26E.

Then, the first variable length portion 122 is changed from the contracted state to the extended state, while the second variable length portion 126 is changed from the extended state to the contracted state (Step S206). Expansion and contraction of the balloons in Step S206 is shown in Fig. 26F.

As illustrated in Fig. 26F, the first variable length portion 122 is changed from the contracted state to the extended state, while the second variable length portion 126 is changed from the extended state to the contracted state. Thus, the propulsion balloon 120 (particularly, with the fixed length of the fixed length portion 124 of the propulsion balloon 120) generates a propulsion force rearward (the black arrow in Fig. 26F) in the advancing direction of the distal end portion 110a and thus generates a force in the advancing direction on the distal end portion 110a.

In Step S204, the propulsion balloon 120 reliably abuts against the intestinal wall 140. Thus, the propulsion force of the propulsion balloon 120 is reliably transferred to the intestinal wall 140, and the force in the advancing direction is reliably generated on the distal end portion 110a.

Thus, as shown by the white arrow in Fig. 26F, the distal end portion 110a of the electronic endoscope 101 moves in the advancing direction relative to the intestinal wall 140.

Next, the gas is filled into the lock balloon 128 to expand the lock balloon 128 and lock the lock balloon 128 on the intestinal wall 140 (Step S207). Expansion and contraction of the balloons in Step S207 is shown in Fig. 26G.

Then, the gas is released from the propulsion balloon 120 to contract the propulsion balloon 120 (Step S208). Expansion and contraction of the balloons in Step S208 is shown in Fig. 26H.

Then, while the first variable length portion 122 is changed from the expanded state to the contracted state, the second variable length portion 126 is changed from the contracted state to the expanded state (Step S209). Expansion and contraction of the balloons returns to the state in Fig. 26A by Step S209.

As such, in Step S208, the propulsion balloon 120 is contracted with the extended state of the first variable length portion 122 and the contracted state of the second variable length portion 126 being maintained, and after the propulsion balloon 120 is contracted to separate the fixed length portion 124 from the intestinal wall 140, in Step S209, the first variable length portion 122 is changed from the extended state to the contracted state, and the second variable length portion 126 is changed from the contracted state to the extended state.

Thus, an unnecessary retraction operation that may be caused by the first variable length portion 122 being changed from the extended state to the contracted state and the second variable length portion 126 being changed from the contracted state to the extended state is not transferred to the intestinal wall 140, and in Step S206, the position of movement of the distal end portion 110a of the electronic endoscope 101 1 in the advancing direction is maintained.

Then, the control flow in Steps S204 to S209 is repeated to continue the propulsion operation of the intraductal moving body actuator.

Then, when an instruction to stop self-propelled movement by the intraductal moving body actuator is given to the electronic endoscope 101 (Step S210), the release of the gas from the propulsion balloon and the lock balloon is started (Step S211). When the release of the gas is finished, that effect is displayed on a display portion (not shown) (Step S212).

The lock balloon 128 and the propulsion balloon 120 may have the same structure and operation.

The above is the description on the propulsion operation flow of the intraductal moving body actuator in Example 1.

### (Example 2)

### <Configuration of intraductal moving body actuator>

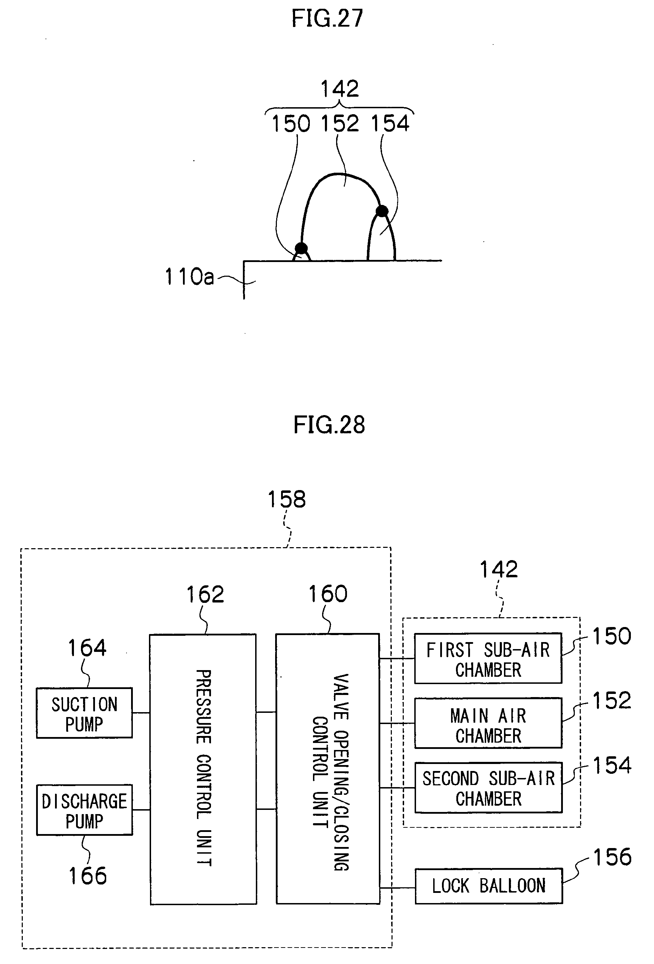

Fig. 27 is an enlarged sectional view of a propulsion balloon as a propulsion mechanism in Example 2 at the distal end portion 110a of the insertion portion 110. As illustrated in Fig. 27, the entire propulsion balloon 142 is made of expandable and contractable latex rubber, and includes three pressure chambers: a main air chamber 152, a first sub-air chamber 150, and a second sub-air chamber 154. The first sub-air chamber 150 and the second sub-air chamber 154 are placed on opposite sides of the main air chamber 152. When the configuration of the propulsion balloon 142 is considered to include a first region and a pair of second regions connected to opposite sides of the first region, an outer peripheral portion of the main air chamber 152 corresponds to the first region, and an outer peripheral portion of the first sub-air chamber 150 and an outer peripheral portion of the second sub-air chamber 154 correspond to the pair of second regions.

When the gas is filled into the three pressure chambers to expand the most the pressure chambers, the main air chamber 152 has a larger volume than volumes of the first sub-air chamber 150 and the second sub-air chamber 154.

Fig. 28 is a block diagram of a balloon control device 158 for controlling pressure of the propulsion balloon 142 and a lock balloon 156. As illustrated in Fig. 28, the propulsion balloon 142 and the lock balloon 156 can be independently adjusted in internal pressure, and the three chambers: the main air chamber 152, the first sub-air chamber 150, and the second sub-air chamber 154 of the propulsion balloon 142 can be also independently adjusted in pressure chamber. A suction pump 164 and a discharge pump 166 are connected to the balloons via a valve opening/closing control unit 160 and a pressure control unit 162.

A propulsion operation flow described below is performed by the valve opening/closing control unit 160 controlling opening/closing of a valve (not shown) connected to each balloon, and the pressure control unit 162 controlling the suction pump 164 and the discharge pump 166.

### <Propulsion operation flow>

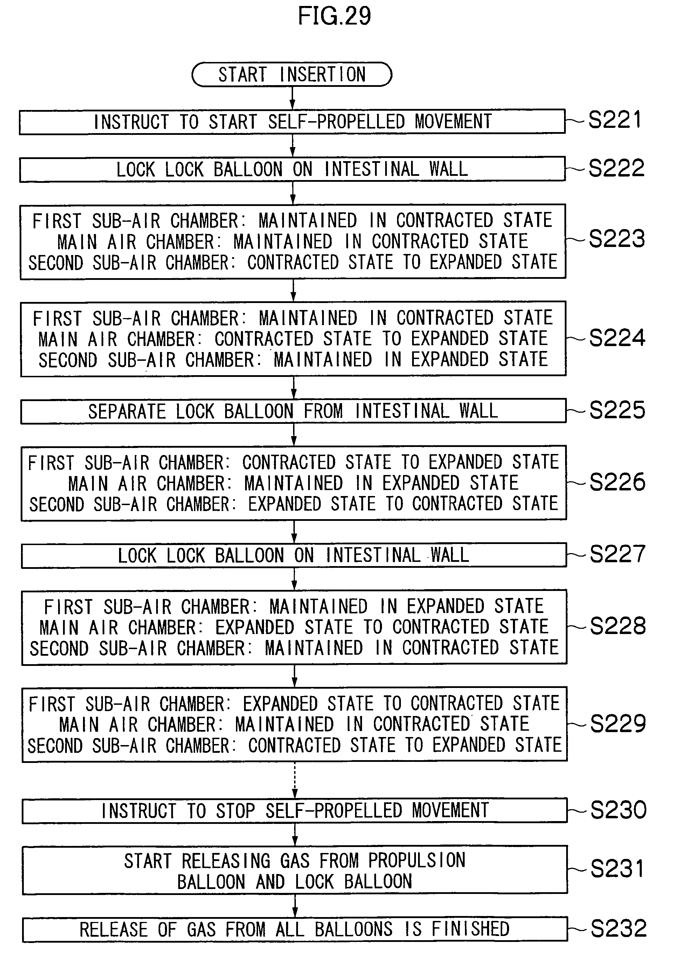

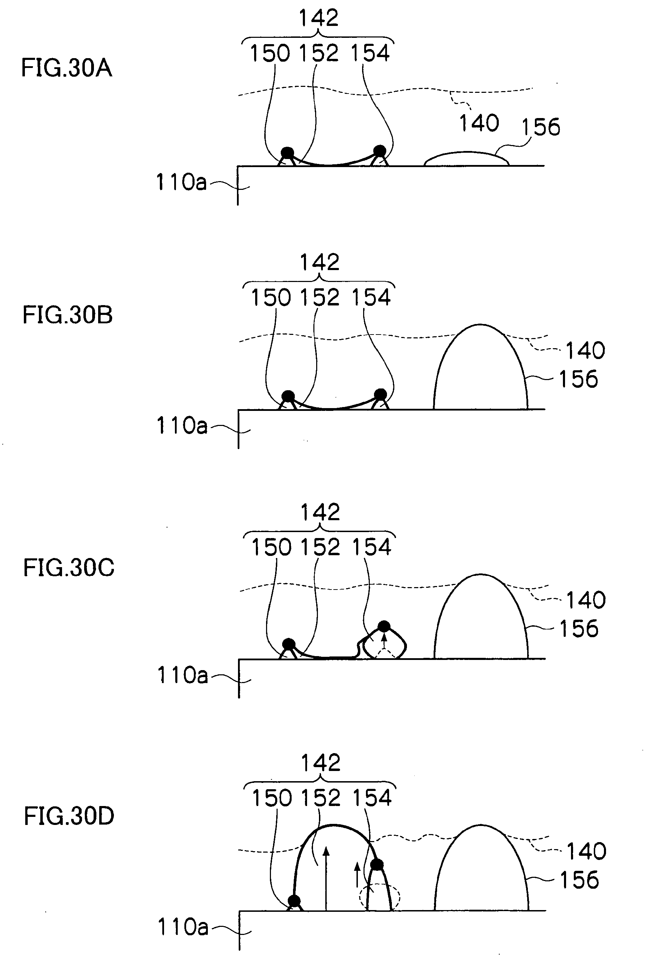

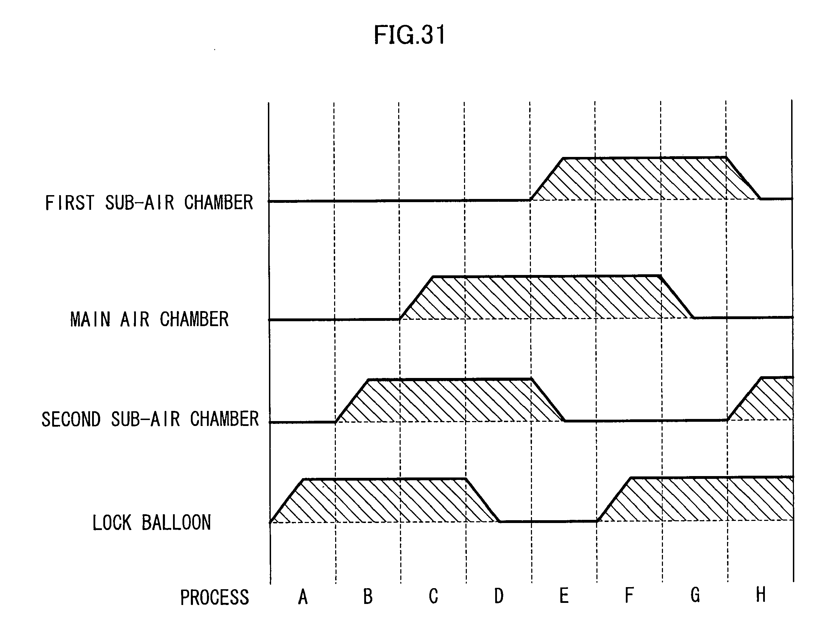

Fig. 29 is a flowchart of a specific propulsion operation of the intraductal moving body actuator of the present invention. Figs. 30A to 30H are schematic sectional views of expansion and contraction of each balloon associated with the flowchart of the propulsion operation in Fig. 29. Fig. 31 is a timing chart illustrating changes with time of pressure in each balloon in the propulsion operation in Fig. 29.

Then, the propulsion operation flow will be described in detail on the basis of Fig. 29 with additional reference to Figs. 30A to 30H and 31.

First, as illustrated in Fig. 30A, with both the propulsion balloon 142 and the lock balloon 156 being contracted (with the pair of second regions corresponding to the outer peripheral portion of the first sub-air chamber 150 and the outer peripheral portion of the second sub-air chamber 154 being contracted), the distal end portion 110a of the electronic endoscope 101 is started to be inserted into a target to be measured (for example, a large intestine), and an instruction to start self-propelled movement by the intraductal moving body actuator is given to the electronic endoscope 101 (Step S221).

Then, gas is filled into the lock balloon 156 to expand the lock balloon 156 and lock the lock balloon 156 on the intestinal wall 140 (Step S222). Expansion and contraction of the balloons in Step S222 is shown in Fig. 30B. Step S222 corresponds to a process A in Fig. 31.

Then, gas is not filled into the main air chamber 152 and the first sub-air chamber 150 of the propulsion balloon 142 and the main air chamber 152 and the first sub-air chamber 150 are maintained in the contracted state, while gas is filled into the second sub-air chamber 154 to expand the second sub-air chamber 154 (Step S223). Expansion and contraction of the balloons in Step S223 is shown in Fig. 30C. As illustrated in Fig. 30C, the second sub-air chamber 154 is expanded, and the second region corresponding to the outer peripheral portion of the second sub-air chamber 154 is extended. Step S223 corresponds to a process B in Fig. 31.

Then, gas is filled until internal pressure of the main air chamber 152 of the propulsion balloon 142 reaches a specified value, and the propulsion balloon 142 is changed from the contracted state to the expanded state (Step S224). Expansion and contraction of the balloons in Step S224 is shown in Fig. 30D. As illustrated in Fig. 30D, the propulsion balloon 142 (particularly, the first region as the outer peripheral portion of the main air chamber 152) abuts against and comes into tight contact with the intestinal wall 140. Step S224 corresponds to a process C in Fig. 31.

The specified value of the internal pressure of the main air chamber 152 of the propulsion balloon 142 is a pressure value when the propulsion balloon 142 is brought into tight contact with the intestinal wall 140 with slack in the intestinal wall 140 being removed, and is a pressure value at which the propulsion balloon 142 does not break the intestinal wall 140 and does not slide on the intestinal wall 140.

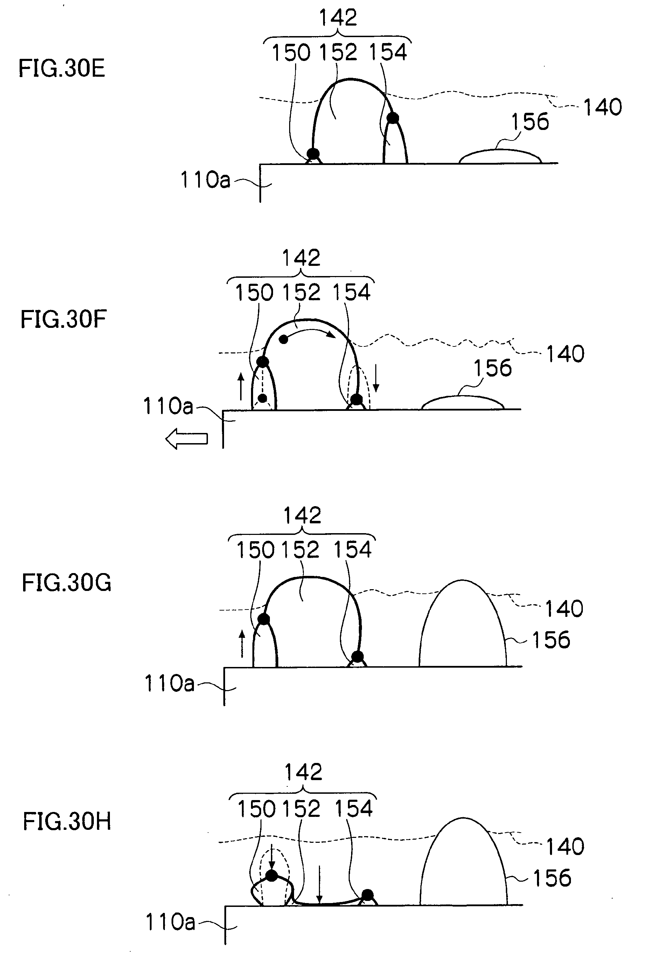

Then, the gas is released from the lock balloon 156 to separate the lock balloon 156 from the intestinal wall 140 (Step S225). Expansion and contraction of the balloons in Step S225 is shown in Fig. 30E. Step S225 corresponds to a process D in Fig. 31.

Then, the gas is filled into the first sub-air chamber 150 of the propulsion balloon 142 to change the first sub-air chamber 150 from the contracted state to the expanded state, while the gas is released from the second sub-air chamber 154 of the propulsion balloon 142 to change the second sub-air chamber 154 from the expanded state to the contracted state (Step S226). Expansion and contraction of the balloons in Step S226 is shown in Fig. 30F. As illustrated in Fig. 30F, the first sub-air chamber 150 is expanded and one of the second regions corresponding to the outer peripheral portion of the first sub-air chamber 150 is extended, while the second sub-air chamber 154 is contracted and the other of the second regions corresponding to the outer peripheral portion of the second sub-air chamber 154 is contracted. Step S226 corresponds to a process E in Fig. 31.

As illustrated in Fig. 30F, the gas is filled into the first sub-air chamber 150, while the gas is released from the second sub-air chamber 154, and thus the propulsion balloon 142 generates a propulsion force rearward (the black arrow in Fig. 30F) in the advancing direction of the distal end portion 110a to generate a force in the advancing direction on the distal end portion 110a.

In Step S224, the propulsion balloon 142 is reliably abutted against the intestinal wall 140, and thus the propulsion force of the propulsion balloon 142 is reliably transferred to the intestinal wall 140 to reliably generate the force in the advancing direction on the distal end portion 110a with respect to the intestinal wall 140. This moves the distal end portion 110a of the electronic endoscope 101 in the advancing direction relative to the intestinal wall 140 as the white arrow in Fig. 30F.

Then, the gas is filled into the lock balloon 156 to expand the lock balloon 156 and lock the lock balloon 156 on the intestinal wall 140 (Step S227). Expansion and contraction of the balloons in Step S227 is shown in Fig. 30G. Step S227 corresponds to a process F in Fig. 31.

Then, the gas is released from the main air chamber 152 of the propulsion balloon 142 to contract the main air chamber 152 (Step S228). Expansion and contraction of the balloons in Step S228 is shown in Fig. 30H. Step S228 corresponds to a process G in Fig. 31.

Then, the gas is released from the first sub-air chamber 150 of the propulsion balloon 142 to change the first sub-air chamber 150 from the expanded state to the contracted state, while the gas is filled into the second sub-air chamber 154 of the propulsion balloon 142 to change the second sub-air chamber 154 from the contracted state to the expanded state (Step S229). Expansion and contraction of the balloons returns to the state in Fig. 30C by Step S229. Step S229 correspond to a process H in Fig. 31.

As such, in Step S228, the main air chamber 152 is contracted with the expanded state of the first sub-air chamber 150 and the contracted state of the second sub-air chamber 154 being maintained, and after the main air chamber 152 is contracted and separated from the intestinal wall 140, in Step S229, the first sub-air chamber 150 is changed from the expanded state to the contracted state and the second sub-air chamber 154 is changed from the contracted state to the expanded state.

Thus, an unnecessary retraction operation that may be caused by the first sub-air chamber 150 being changed from the expanded state to the contracted state and the second sub-air chamber 154 being changed from the contracted state to the expanded state is not transferred to the intestinal wall 140, and in Step S226, the position of movement of the distal end portion 110a of the electronic endoscope 101 in the advancing direction is maintained.

Then, the control flow in Step S224 to S229 is repeated to continue the propulsion operation of the intraductal moving body actuator.

Then, when an instruction to stop self-propelled movement by the intraductal moving body actuator is given to the electronic endoscope 101 (Step S230), the release of the gas from the first sub-air chamber 150, the main air chamber 152, and the second sub-air chamber 154 of the propulsion balloon 142 and the lock balloon 156 is started (Step S231). When the release of the gas is finished, that effect is displayed on a display portion (not shown) (Step S232).

The lock balloon 156 and the propulsion balloon 142 may have the same structure and operation.

The above is the description on the propulsion operation flow of the intraductal moving body actuator in Example 2.

### (Example 3)

### <Configuration of intraductal moving body actuator>

Figs. 32A and 32B illustrate a configuration of an intraductal moving body actuator in Example 3 at the distal end portion 110a of the insertion portion 110. Fig. 32A is a schematic view seen from a lateral side of the distal end portion 110a, and Fig. 32B is a view seen from a distal end side of the distal end portion 110a.

For convenience of description, Fig. 32A illustrates a first propulsion balloon 172 only in sectional view.

As illustrated in Figs. 32A and 32B, the intraductal moving body actuator includes first lift-up balloons 170 that are provided on an outer peripheral surface of the distal end portion 110a of the electronic endoscope 101 and lifts up a first propulsion balloons 172 described below, and the first propulsion balloons 172 provided to overlap an upper side (outer peripheral side) of the first lift-up balloons 170.

The first lift-up balloons 170 serve as a base mechanism that support the first propulsion balloons 172 and cause the first propulsion balloons 172 to abut against and separate from a duct wall such as an intestinal wall. The first propulsion balloons 172 serve as a propulsion mechanism that generate a drive force via the duct wall such as the intestinal wall to generate a propulsion force for movement in a duct.

An interface between the first lift-up balloon 170 and the first propulsion balloon 172 is connected by bonding or integral molding.

A pair of bilayer structure balloons as bilayer mechanisms each including the first lift-up balloon 170 and the first propulsion balloon 172 are arranged axially symmetrically at the distal end portion 110a. In the embodiment, a pair of bilayer structure balloons as bilayer mechanisms each including a second lift-up balloon 174 and a second propulsion balloon 176 are additionally arranged axially symmetrically at the distal end portion 110a of the endoscope in circumferentially 90° shifted positions on the distal end portion 10a. Thus, two pairs of bilayer structure balloons are provided, in total. A total of three or more pairs of bilayer structure balloons may be provided.

In the embodiment, the two pairs of bilayer structure balloons are provided substantially in the same axial positions on the distal end portion 110a of the electronic endoscope 101, but one pair of bilayer structure balloons and the other pair of bilayer structure balloons may be provided in axially shifted positions.

The first propulsion balloons 172 and the second propulsion balloons 176 respectively have the same structure and operation as those of the propulsion balloons 120 (see Fig. 23) in Example 1. The first propulsion balloons 172 and the second propulsion balloons 176 may have the same structure and operation as those of the propulsion balloons 142 (see Fig. 27) in Example 2, but descriptions will be herein made with the propulsion balloons 120 in Example 1.

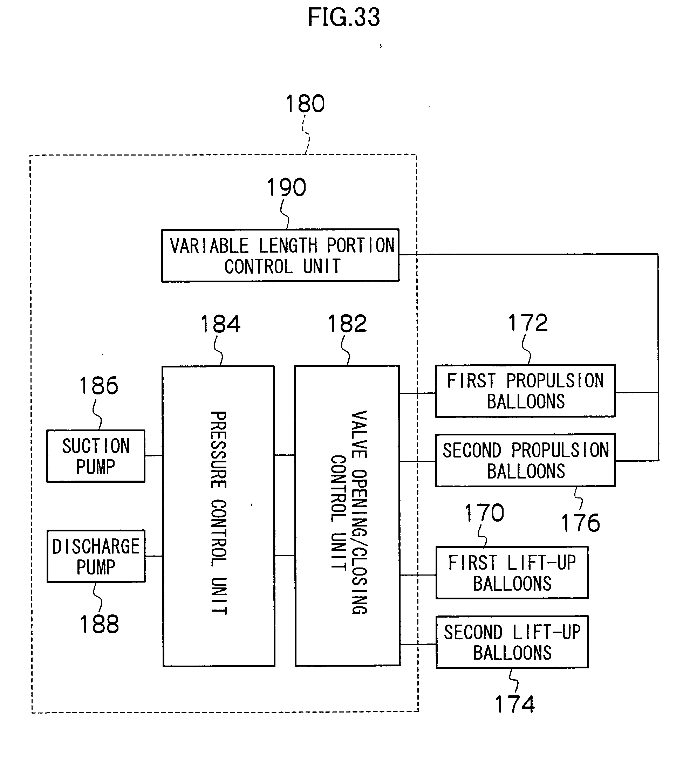

Fig. 33 is a block diagram of a balloon control device 180 for controlling pressure of the first lift-up balloons 170, the first propulsion balloons 172, the second lift-up balloons 174, and the second propulsion balloons 176, and controlling expansion and contraction of first variable length portions 122 and second variable length portions 126 of respective first propulsion balloons 172 and second propulsion balloons 176. As illustrated in Fig. 33, the first lift-up balloons 170, the first propulsion balloons 172, the second lift-up balloons 174, and the second propulsion balloons 176 can be independently adjusted in internal pressure, and a suction pump 186 and a discharge pump 188 are connected to the balloons via a valve opening/closing control unit 182 and a pressure control unit 184.

The balloon control device 180 also includes a variable length portion control unit 190 for controlling the first variable length portions 122 and the second variable length portions 126 of respective first propulsion balloons 172 and second propulsion balloons 176.

A propulsion operation flow described below is performed by the valve opening/closing control unit 182 controlling opening/closing of a valve (not shown) connected to each balloon, the pressure control unit 184 controlling the suction pump 186 and the discharge pump 188, and the variable length portion control unit 190 heating or cooling the first variable length portions 122 and the second variable length portions 126 of respective first propulsion balloons 172 and second propulsion balloons 176 so that the variable length portions are contracted or extended.

### <Propulsion operation flow>

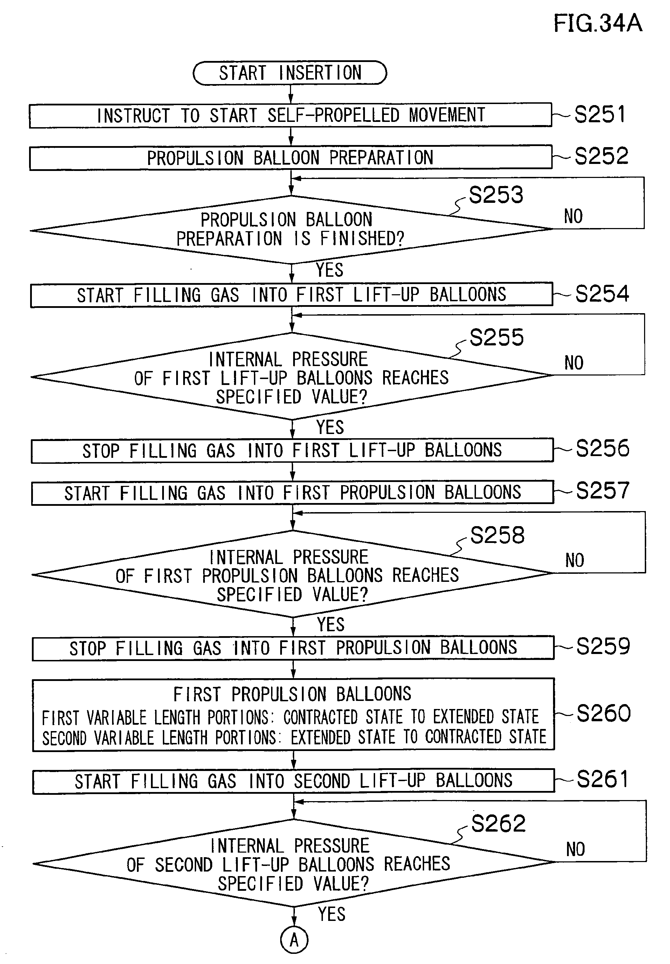

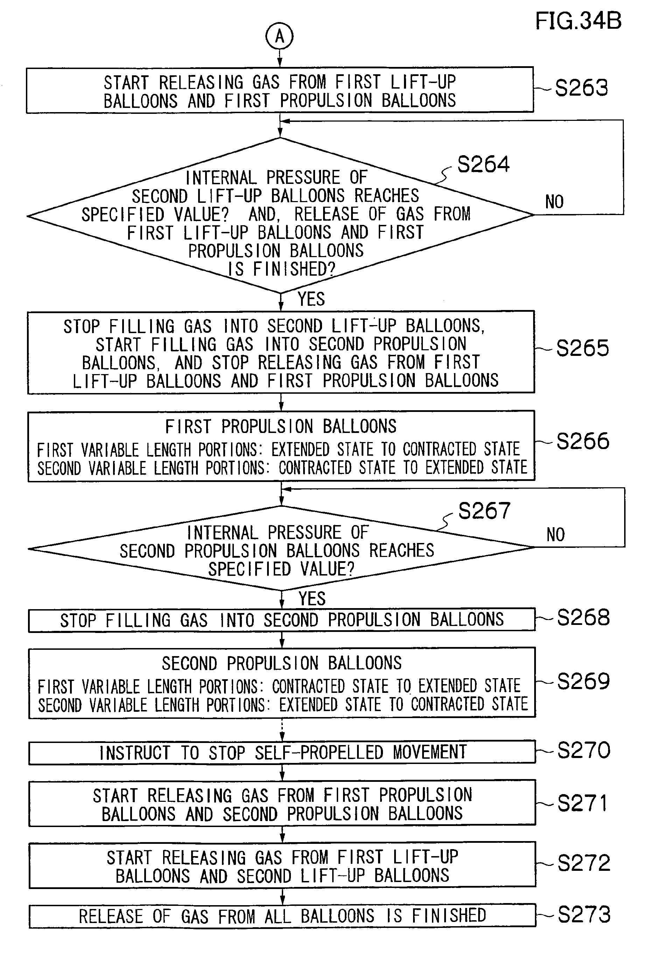

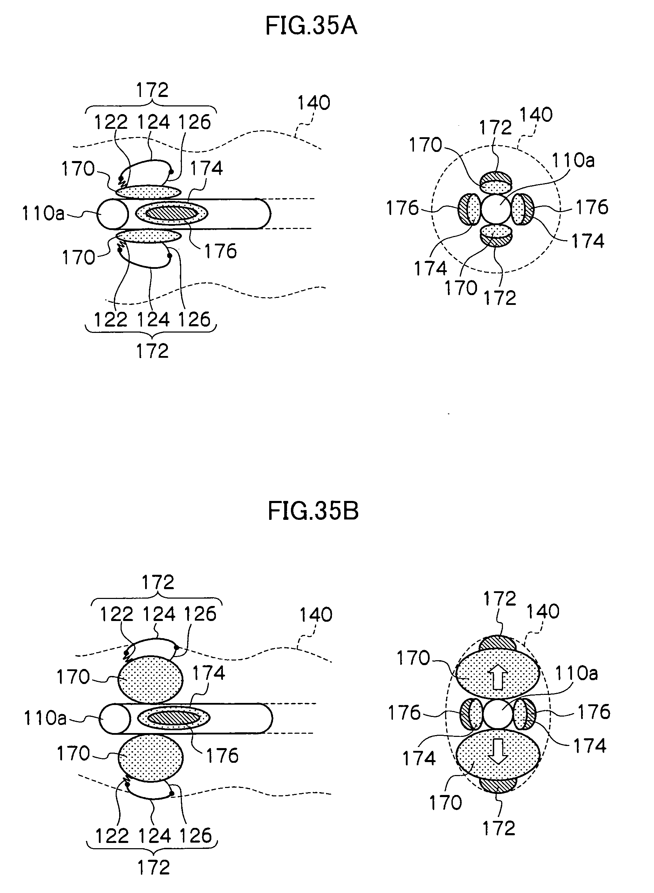

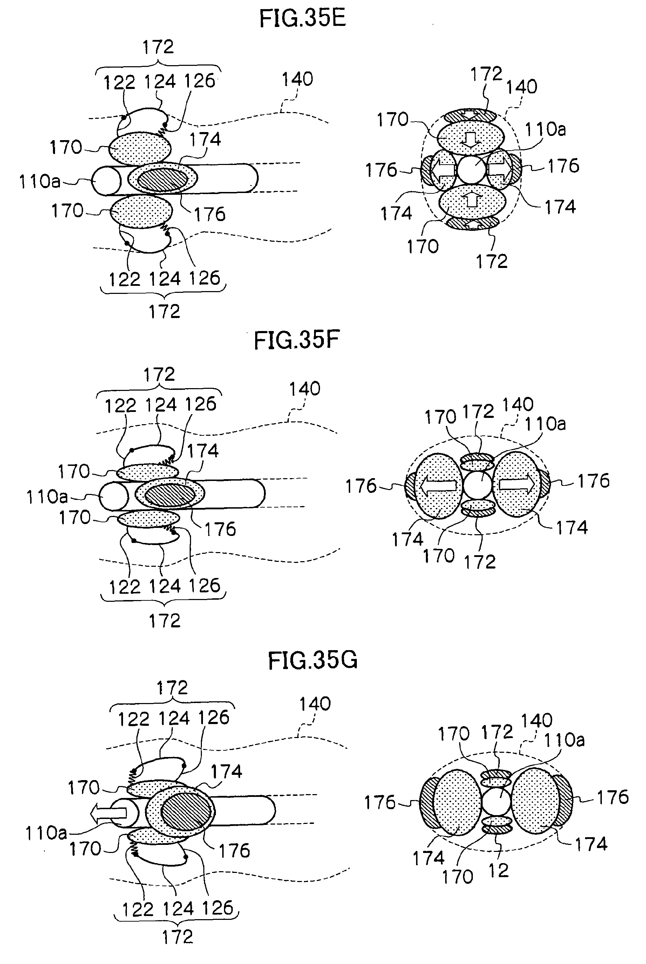

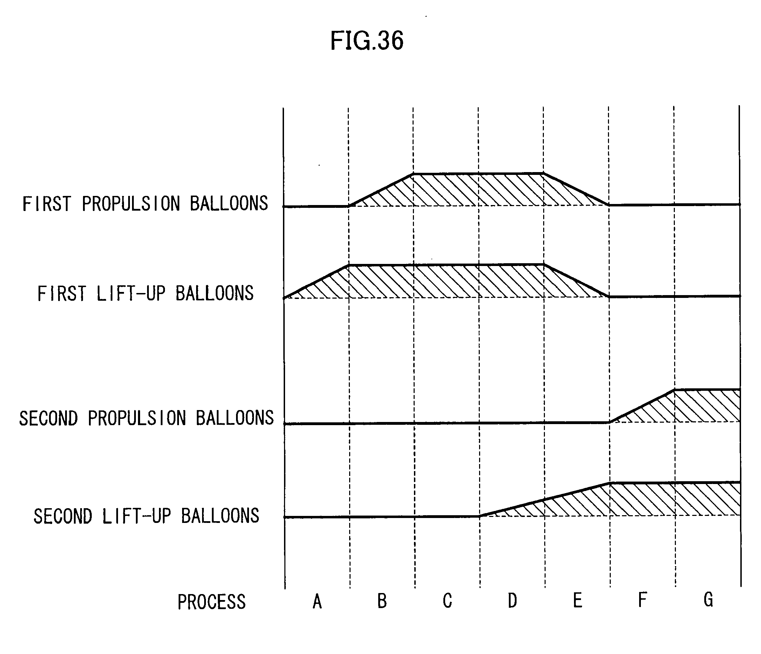

Figs. 34A and 34B are a flowchart of a detailed propulsion operation of the intraductal moving body actuator of the present invention. Figs. 35A to 35G are schematic views of expansion and contraction of each balloon associated with the flowchart of the propulsion operation in Figs. 34A and 34B, left views in Figs. 35A to 35G are schematic views seen from a lateral side of the distal end portion 110a, and right views in Figs. 35A to 35G are views seen from a distal end side of the distal end portion 110a. For convenience of description, the left views in Figs. 35A to 35G illustrate the first propulsion balloon 172 only in sectional view. Fig. 36 is a timing chart illustrating changes with time of pressure in each balloon in the propulsion operation in Figs. 34A and 34B.

Then, the propulsion operation flow will be described in detail on the basis of Figs. 34A and 34B with additional reference to Figs. 35A to 35G and 36.

As illustrated in Figs. 34A and 34B, the distal end portion 110a of the electronic endoscope 101 is started to be inserted into a target to be measured (for example, a large intestine), and an instruction to start self-propelled movement by the intraductal moving body actuator is given to the electronic endoscope 101 (Step S251).

Then, as propulsion balloon preparation, with the contracted states of the first propulsion balloons 172 and the second propulsion balloons 176 being maintained, the contracted state of the first variable length portions 122 of respective first propulsion balloons 172 and second propulsion balloons 176 is maintained, and the second variable length portions 126 are extended (Step S252). Expansion and contraction of each balloon in Step S252 is shown in Fig. 35A.

When the propulsion balloon preparation is finished (Step S253), then filling of gas into the first lift-up balloons 170 is started (Step S254).

When internal pressure of the first lift-up balloons 170 reaches a specified value (Step S255), the filling of the gas into the first lift-up balloons 170 is stopped (Step S256). The specified value of the internal pressure of the first lift-up balloons 170 is a pressure value when the first propulsion balloons 172 (particularly, the fixed length portions 124 of the first propulsion balloons 172) are brought into tight contact with the intestinal wall 140 with slack in an intestinal wall 140 being removed, and is a pressure value at which the first propulsion balloons 172 do not break the intestinal wall 140 and do not slide on the intestinal wall 140.

Expansion and contraction of each balloon in Step S256 is shown in Fig. 35B. As illustrated in Fig. 35B, the gas is filled into the first lift-up balloons 170 to lift up the first propulsion balloons 172, and the first propulsion balloons 172 abut against and come into tight contact with the intestinal wall 140. A circumferential length of the intestinal wall 140 does not change, and the intestinal wall 140 is completely stretched in a direction of the first propulsion balloons 172 being lifted up. Thus, the first propulsion balloons 172 come into tight contact with the intestinal wall 140 with the slack in the intestinal wall 140 being removed. The second propulsion balloons 176 are separated from the intestinal wall 140.

At this time (Steps S254 to S256), a timing chart of each balloon corresponds to a process A in Fig. 36.

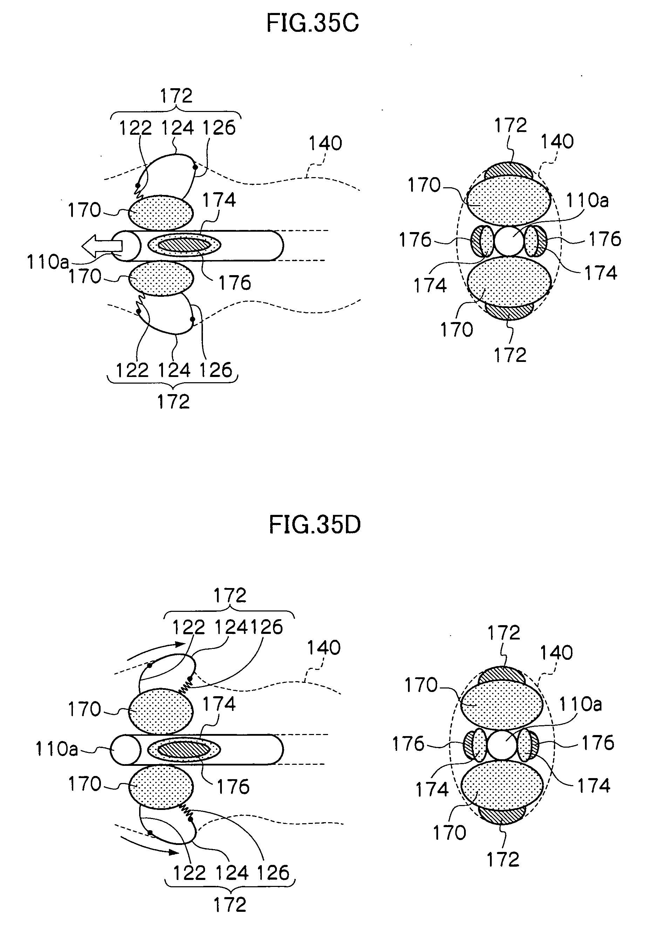

Then, the filling of the gas into the first propulsion balloons 172 is started (Step S257), and when the internal pressure of the first propulsion balloons 172 reaches a specified value (Step S258), the filling of the gas into the first propulsion balloons 172 is stopped (Step S259). Expansion and contraction of each balloon in Step S259 is shown in Fig. 35C.

The specified value of the internal pressure of the first propulsion balloons 172 is a pressure value when the first propulsion balloons 172 are brought into tighter contact with the intestinal wall 140 to more reliably remove slack in the intestinal wall 140 than when the first lift-up balloons 170 cause the first propulsion balloons 172 in the contracted state to abut against and to be brought into tight contact with the intestinal wall 140 in Step S256, and is a pressure value at which the first propulsion balloons 172 do not break the intestinal wall 140 and do not slide on the intestinal wall 140.

The first lift-up balloons 170 cause the first propulsion balloons 172 in the contracted state to previously abut against the intestinal wall 140 in Step S256, and thus as compared with Example 1, the first propulsion balloons 172 are brought into tight contact with the intestinal wall 140 while slack in the intestinal wall 140 are more reliably removed, even with a smaller amount of gas filled into the first propulsion balloons 172.

At this time (Step S257 to S259), a timing chart of each balloon corresponds to a process B in Fig. 36.

Then, while the first variable length portions 122 of the first propulsion balloons 172 change from the contracted state to the extended state, the second variable length portions 126 change from the extended state to the contracted state (Step S260).

Expansion and contraction of each balloon of Step S260 is shown in Fig. 35D. As illustrated in Fig. 35D, the first propulsion balloons 172 generate a propulsion force rearward (the black arrow in Fig. 35D) in the advancing direction of the distal end portion 110a, and thus a force in the advancing direction is generated on the distal end portion 110a.

The first propulsion balloons 172 reliably abut against the intestinal wall 140 in Step S256, and thus the gas is filled into the first propulsion balloons 172 to reliably generate the force in the advancing direction on the distal end portion 110a. Thus, as the white arrow in Fig. 35D, the distal end portion 110a of the electronic endoscope 101 moves in the advancing direction relative to the intestinal wall 140.

At this time (S260), a timing chart of each balloon corresponds to a process C in Fig. 36.

Then, filling of the gas into the second lift-up balloons 174 is started (Step S261).

When the internal pressure of the second lift-up balloons 174 reaches a predetermined passing value (Step S262), the second propulsion balloons 176 abut against the intestinal wall 140, and at this time, release of the gas from the first lift-up balloons 170 and the first propulsion balloons 172 is started (Step S263).

The predetermined passing value of the internal pressure of the second lift-up balloons 174 is a passing value which is present before the second lift-up balloons 174 expand and its internal pressure reaches a specified value, and is a value of the internal pressure of the second lift-up balloons 174 when the second propulsion balloons 176 abut against the intestinal wall 140.

Expansion and contraction of each balloon in Step S263 is shown in Fig. 35E. As illustrated in Fig. 35E, the gas is filled into the second lift-up balloons 174 to expand the second lift-up balloons 174 and thus lift up the second propulsion balloons 176 in the direction of the white arrow. When the second propulsion balloons 176 abut against the intestinal wall 140, the first lift-up balloons 170 and the first propulsion balloons 172 are contracted in the direction of the white arrow while the extended state of the first variable length portions 122 and the contracted state of the second variable length portions 126 of the first propulsion balloons 172 are maintained.

As such, the first lift-up balloons 170 and the first propulsion balloons 172 are contracted while the expanded state of the first variable length portions 122 and the contracted state of the second variable length portions 126 of the first propulsion balloons 172 are maintained, and thus an unnecessary retraction operation is not transferred to the intestinal wall 140, and in Step S260, the position of movement of the distal end portion 110a of the electronic endoscope 101 in the advancing direction is maintained.

Also, the first propulsion balloons 172 are separated from the intestinal wall 140 after the second propulsion balloons 176 are abutted against the intestinal wall 140, and thus even if the first propulsion balloons 172 are separate from the intestinal wall 140, the distal end portion 110a is locked on the intestinal wall 140 by the second propulsion balloons 176 to maintain its position.

At this time (Step S261 to S263), a timing chart of each balloon corresponds to a process D in Fig. 36.

Then, when the internal pressure of the second lift-up balloons 174 passes the predetermined passing value and reaches the specified value, and the release of the gas from the first lift-up balloons 170 and the first propulsion balloons 172 is finished (Step S264), the filling of the gas into the second lift-up balloons 174 is stopped, while the filling of the gas into the second propulsion balloons 176 is started, and the release of the gas from the first lift-up balloons 170 and the first propulsion balloons 172 is stopped (Step S265).

The specified value of the internal pressure of the second lift-up balloons 174 is a pressure value when the second propulsion balloons 176 are brought into tight contact with the intestinal wall 140 with slack in the intestinal wall 140 being removed, and is a pressure value at which the second propulsion balloons 176 do not break the intestinal wall 140 and do not slide on the intestinal wall 140.

At this time, expansion and contraction of each balloon is shown in Fig. 36F. As illustrated in Fig. 36F, the first propulsion balloons 172 are separated from the intestinal wall 140 with the extended state of the first variable length portions 122 and the contracted state of the second variable length portions 126 being maintained.

Meanwhile, the gas is filled into the second lift-up balloons 174 to lift up the second propulsion balloons 176, and thus the second propulsion balloons 176 abut against and come into contact with the intestinal wall 140. A circumferential length of the intestinal wall 140 does not change, and the intestinal wall 140 is completely stretched in a direction of the second propulsion balloons 176 being lifted up. Thus, the second propulsion balloons 176 come into tight contact with the intestinal wall 140 with the slack in the intestinal wall 140 being removed.

At this time (Steps S264 and S265), a timing chart of each balloon corresponds to a process E in Fig. 36.

Then, the first variable length portions 122 of the first propulsion balloons 172 change from the extended state to the contracted state, and the second variable length portions 126 change from the contracted state to the extended state (Step S266).

Then, when the internal pressure of the second propulsion balloons 176 reaches the specified value (Step S267), the filling of the gas into the second propulsion balloons 176 is stopped (Step S268).

Expansion and contraction of each balloon in Step S268 is shown in Fig. 35G.

At this time (Step S266 to S268), a timing chart of each balloon corresponds to a process F in Fig. 36.

Then, the first variable length portions 122 of the second propulsion balloons 176 change from the contracted state to the extended state, while the second variable length portions 126 of the second propulsion balloons 176 change from the extended state to the contracted state (Step S269). Thus, as in Step S260, the second propulsion balloons 176 generate a propulsion force rearward in the advancing direction of the distal end portion 110a to generate a force in the advancing direction on the distal end portion 110a.

At this time (Step S269), a timing chart of each balloon corresponds to a process G in Fig. 36.

Then, the process returns to Step S261, and thereafter, the first lift-up balloons 170 and the second lift-up balloons 174 are interchanged, and the first propulsion balloons 172 and the second propulsion balloons 176 are interchanged to perform the same flow, and the flow is repeated to continue the propulsion operation of the intraductal moving body actuator. Detailed descriptions thereof will be omitted because of its overlapping contents.

Then, when an instruction to stop self-propelled movement by the intraductal moving body actuator is given to the electronic endoscope 101 (Step S270), the release of the gas from the first propulsion balloons 172 and the second propulsion balloons 176 is started (Step S271), and the release of the gas from the first lift-up balloons 170 and the second lift-up balloons 174 is started (Step S272). When the release of the gas from all the balloons is finished, that effect is displayed on a display portion (not shown) (Step S273).

In Step S263, the gas is simultaneously released from the first lift-up balloons 170 and the first propulsion balloons 172, but not limited thereto, it may be allowed that the gas is released from the first lift-up balloons 170 only, and the first propulsion balloons 172 are separated from the intestinal wall 140 while the first propulsion balloons 172 are maintained in the expanded state, the first variable length portions 122 are maintained in the extended state, and the second variable length portions 126 are maintained in the contracted state.

The above is the description on the propulsion operation flow.

As described above on the propulsion operation, the first propulsion balloons 172 (or second propulsion balloons 176) in the contracted state abut against the intestinal wall 140, then the first variable length portions 122 change from the contracted state to the extended state, and the second variable length portions 126 change from the extended state to the contracted state to apply the propulsion force to the intestinal wall 140, while the first propulsion balloons 172 (or second propulsion balloons 176) are separate from the intestinal wall 140 while the extended state of the first variable length portions 122 and the contracted state of the second variable length portions 126 of the first propulsion balloons 172 (or second propulsion balloons 176) are maintained.

As such, the state of the first propulsion balloons 172 (or second propulsion balloons 176) when they abut against the intestinal wall 140, and the state of the first propulsion balloons 172 (or second propulsion balloons 176) when they separate from the intestinal wall 140 are made different to provide so-called hysteresis to the state of the first propulsion balloons 172 (or second propulsion balloons 176) when they abut against and separate from the intestinal wall 140.

Thus, an unnecessary retraction operation is not transferred to the intestinal wall 140, and thus an amount of movement can be maintained of the distal end portion 110a of the electronic endoscope 101 advanced by the propulsion force generated when the first propulsion balloons 172 (or second propulsion balloons 176) abut against the intestinal wall 140, and a large amount of movement can be obtained in the advancing direction.

The first lift-up balloons 170 (or second lift-up balloons 174) are expanded to cause the first propulsion balloons 172 (or second propulsion balloons 176) to reliably abut against the intestinal wall 140, thereby the propulsion force from the first propulsion balloons 172 (or second propulsion balloons 176) to be reliably transferred to the intestinal wall 140.

### (Variant)

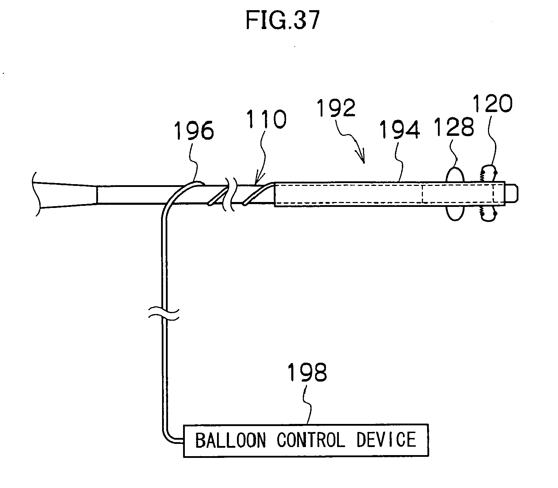

In the above embodiment, the example in which the balloons are directly mounted to the insertion portion 110 of the electronic endoscope 101 is described. The present invention is not limited thereto, but may be applied to an endoscope moving device 192 in Fig. 37.

The endoscope moving device 192 includes a cylinder 194 in which the insertion portion 110 is inserted and secured, any of the propulsion balloon 120 and the lock balloon 128, the propulsion balloon 142 and the lock balloon 128, and the bilayer structure balloons mounted to a distal end of the cylinder 194, and a balloon control device 198 having the same configuration as any of the balloon control devices 118, 158 and 180 corresponding to the balloons mounted to the distal end of the cylinder 194 and to which a cord 196 extending from the cylinder 194 is connected.

As in the above embodiment, a bilayer structure balloons are arranged axially symmetrically, and a pair of bilayer structure balloons are arranged axially symmetrically in circumferentially 90° shifted positions. Thus, two pairs of bilayer structure balloons are provided, in total. A total of three or more pairs of bilayer structure balloons may be provided.

When the insertion portion 10 is inserted into a subject, the cylinder 194 is inserted into and secured in the insertion portion 110, and the balloon control device 198 performs the same control as in the above embodiment to move the insertion portion 110.

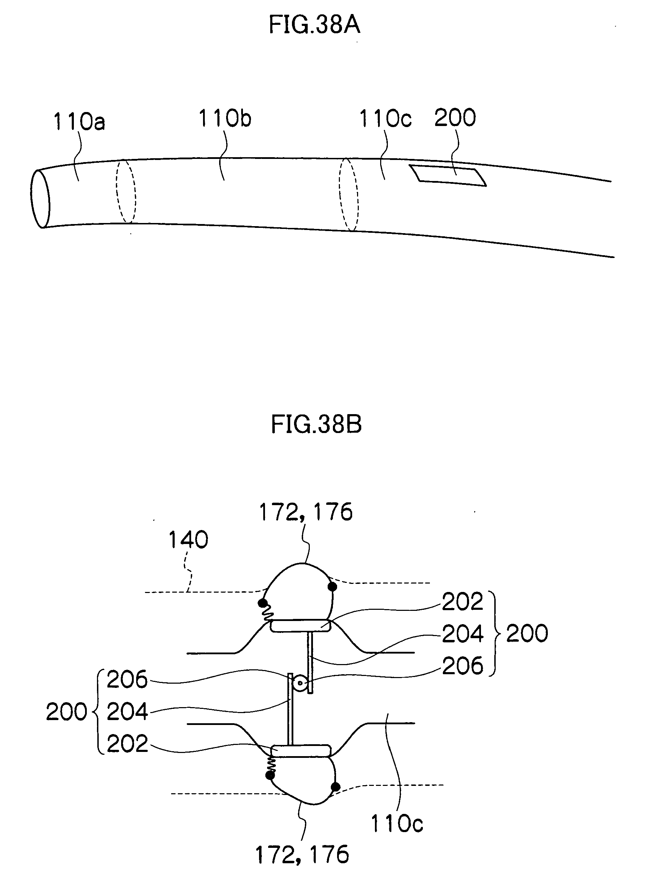

In Example 3, the example in which the balloons are used as a base mechanism at the distal end portion 110a (see Fig. 32) is described. Alternatively, for example, as illustrated in Fig. 38, a lift mechanism 200 as a base mechanism may be provided on a flexible portion 110c, which has relatively more space for arranging components. For convenience of description, first propulsion balloons 172 or second propulsion balloons 176 as a propulsion mechanism is omitted in Fig. 38A.

As illustrated in Fig. 38B, a pair of lift mechanisms 200 are provided axially symmetrically on the flexible portion 110c, and each include a sheet-like rigid pad 202, a first gear 204 connected to the pad 202, and a second gear 206 that meshes with the first gear 204 and including a drive motor (not shown). The second gear 206 is shared by the pair of lift mechanisms 200. Though not shown in Fig. 38, another pair of lift mechanisms 200 are provided in circumferentially 90° shifted positions in an axially shifted position on the flexible portion 110c.

The lift mechanism 200 rotates the second gear 206 with a motor to vertically move the first gear 204 to vertically move the pad 202, and causes the first propulsion balloons 172 or the second propulsion balloons 176 as the propulsion mechanism to be abutted against and separated from the intestinal wall 140.

As described above, the intraductal moving body actuator and the endoscope of the present invention are described in detail, but the present invention is not limited to the above examples, and it should be understood that various changes or modifications may be made without departing from the scope of the present invention.

## Claims

1. An actuator for intraductal moving body, comprising:
a propulsion mechanism (32, 36, 54, 56) which generates a drive force via a duct wall (52) to generate a propulsion force for movement in a duct;
a base mechanism (30, 34, 80) which supports the propulsion mechanism and causes the propulsion mechanism to abut against and separate from the duct wall; and
two or more pairs of bilayer mechanisms provided axially symmetrically.

2. An actuator for intraductal moving body, comprising:
a propulsion mechanism (32, 36, 54, 56) which generates a drive force via a duct wall (52) to generate a propulsion force for movement in a duct;
a base mechanism (30, 34, 80) which supports the propulsion mechanism and causes the propulsion mechanism to abut against and separate from the duct wall;
a pressure source (40, 42) connected to the base mechanism; and
a valve (38, 45) which is provided between the propulsion mechanism and the base mechanism, the valve which is closed until a first pressure difference value is reached and is opened when the first pressure difference value is reached in the case where a pressure in the base mechanism becomes positive with respect to the propulsion mechanism and pressure difference between the propulsion mechanism and the base mechanism is increased by the pressure source, and which is closed until a second pressure difference value is reached and is opened when the second pressure difference value is reached in the case where the pressure in the base mechanism becomes negative with respect to the propulsion mechanism and pressure difference between the propulsion mechanism and the base mechanism is increased by the pressure source.

3. The actuator for intraductal moving body according to claim 1 or 2, further comprising
a control unit (18) which performs control to make the propulsion mechanism generate the propulsion force after the propulsion mechanism is caused to abut against the duct wall by the base mechanism, and to make the base mechanism separate the propulsion mechanism from the duct wall while the propulsion mechanism generating the propulsion force.

4. The actuator for intraductal moving body according to any one of claims 1 to 3, wherein the base mechanism includes one or more balloons, and
the one or more balloons are expanded and contracted to cause the propulsion mechanism to be abut against and separate from the duct wall.

5. The actuator for intraductal moving body according to any one of claims 1 to 4, wherein the propulsion mechanism includes one or more balloons, and
each of the one or more balloons comprises a portion having a lower expansion coefficient than other portions is provided at least in a rear portion in an advancing direction.

6. The actuator for intraductal moving body according to claim 5, wherein the base mechanism causes the one or more balloons in a contracted state to abut against the duct wall, and then the base mechanism causes the one or more balloons in an expanded state to separate from the duct wall.

7. An endoscope (1) having a function of self-propelled movement in a duct and comprising
the actuator for intraductal moving body according to any one of claims 1 to 6.

8. An actuator for intraductal moving body, comprising:
a propulsion mechanism (120, 142) having a first region (124, 152) and a pair of second regions (122, 126, 150, 154) connected to opposite ends of the first region and applying a propulsion force to a duct wall (52, 140); and
a control unit (118, 158) which performs control so that one of the second regions is changed from a contracted state to an extended state and the other of the second regions is changed from the extended state to the contracted state with the first region abutting against the duct wall, in order to apply the propulsion force with respect to the duct wall to the first region applies.

9. The actuator for intraductal moving body according to claim 8, wherein the control unit (118, 158) controls to maintain the one of the second regions in the extended state and maintain the other of the second regions in the contracted state in order to make the first region separate from the duct wall while a state where the propulsion force to the duct wall is applied to the first region being maintained.

10. The actuator for intraductal moving body according to claim 8 or 9, wherein the one of the second regions (122, 150), the first region (124, 152), and the other of the second regions (126, 154) are arranged in order in a movement direction.

11. The actuator for intraductal moving body according to any one of claims 8 to 10, wherein the second region includes shape memory material or artificial muscle.

12. The actuator for intraductal moving body according to any one of claims 8 to 10, wherein the second region (150, 154)includes an outer peripheral portion of a pressure chamber (152), and
the control unit (158) controls to change an internal pressure of the pressure chamber to extend or contract the second region.

13. The actuator for intraductal moving body according to any one of claims 8 to 12, further comprising
a locking mechanism (128, 156) which locks on the duct wall.

14. The actuator for intraductal moving body according to claim 13, wherein the locking mechanism (128, 156) includes the first region and the pair of second regions.

15. The actuator for intraductal moving body according to any one of claims 8 to 14, further comprising
a base mechanism (170, 174) which supports the propulsion mechanism (172, 176) and causes the propulsion mechanism to abut against and separate from the duct wall.

16. An endoscope (101) having a function of self-propelled movement in a duct, comprising an intraductal moving body actuator according to any one of claims 8 to 15.
